# EUROPEAN PATENT SPECIFICATION

(11) **EP 0 843 725 B1**
(45) Date of publication and mention of the grant of the patent: **17.04.2002**
(21) Application number: 96926325.0
(22) Date of filing: 12.08.1996
(51) Int. Cl.: C12N 15/10, C12N 15/62, C12N 15/63

(54) **METHOD FOR PREPARING POLYPEPTIDE VARIANTS**
VERFAHREN ZUR HERSTELLUNG VON POLYPEPTIDABKÖMMLINGEN
PROCEDER POUR PREPARER DES VARIANTS POLYPEPTIDIQUES

(30) Priority: 11.08.1995 DK 90795; 20.09.1995 DK 104795
(43) Date of publication of application: 27.05.1998
(60) Divisional application: 02002150.7
(73) Proprietor: Novozymes A/S, 2880 Bagsvaerd (DK)
(72) Inventor: OKKELS, Jens, Sigurd, DK-2880 Bagsvaerd (DK)
(86) International application number: DK9600343
(87) International publication number: WO9707205

(56) References cited:
- EP-A- 0 141 484
- US-A- 5 093 257
- GENE, Volume 83, 1989, DENIS POMPON et al., "Protein Engineering by cDNA Recombination in Yeasts: Shuffling of Mammalian Cytochrome P-450 Functions", pages 15-24.

## Description

### FIELD OF THE INVENTION

The present invention relates to a method for preparing polypeptide variants by *in vivo* recombination.

### BACKGROUND OF THE INVENTION

The advantages of producing biologically active polypeptides by cloning naturally occurring DNA sequences from microorganisms, such as fungal organisms and bacteria using recombinant DNA technology have been known for quite some years.

Preparation of novel polypeptide variants and mutants, such as novel modified enzymes with altered characteristics, *e.g.* specific activity, substrate specificity, pH-optimum, pI, Kₘ, Vₘₐₓ etc., have especially during the recent years diligently and successfully been used for obtaining polypeptides with improved properties.

For instance, within the technical field of enzymes the washing and/or dishwashing performance of *e.g.* proteases, lipases, amylases and cellulases have been improved significantly.

In most cases these improvements have been obtained by site-directed mutagenesis resulting in substitution, deletion or insertion of specific amino acid residues which have been chosen either on the basis of their type or on the basis of their location in the secondary or tertiary structure of the mature enzyme (see for instance US patent no. 4,518,584).

An alternative general approach for modifying proteins and enzymes have been based on random mutagenesis, for instance, as disclosed in US 4,894,331 and WO 93/01285

As it is a cumbersome and time consuming process to obtain polypeptide variants or mutants with improved functional properties a few alternative methods for rapid preparation of modified polypeptides have been suggested.

Weber et al., (1983), Nucleic Acids Research, vol 11, 5661-5661, describes a method for modifying genes by *in vivo* recombination between to homologous genes. A linear DNA sequence comprising a plasmid vector flanked to a DNA sequence encoding alpha-1 human interferon in the 5'-end and a DNA sequence encoding alpha-2 human interferon in the 3'-end is constructed and transfected into a rec A positive strain of *E. coli.* Recombinants were identified and isolated using a resistance marker.

Pompon el al., (1989), Gene 83, p. 15-24, describes a method for shuffling gene domains of mammalian cytochrome P-450 by *in vivo* recombination of partially homologous sequences in *Saccharomyces cerevisiae* by transforming *Saccharomyces cerevisia* with a linearized plasmid with filled-in ends, and a DNA fragment being partially homologous to the ends of said plasmid.

Stemmer, (1994), Proc. Natl. Acad. Sci. USA, Vol. 91, 10747-10751; Stemmer, (1994), Nature, vol. 370, 389- 391, concern methods for shuffling homologous DNA sequences by an in *vitro* PCR method. One cycle of shuffling consists of digesting a pool of homologous genes with DNase I. The resulting small fragments are reassembled into full-length genes. Positive recombinant genes containing shuffled DNA sequences are selected from a DNA library based on their improved function. Positive recombinants can be used as the starting material for (an)other shuffling round(s).

US patent no. 5,093,257 (Assignee: Genencor Int. Inc.) discloses a method for producing hybrid polypeptides by *in vivo* recombination. Hybrid DNA sequences are produced by forming a circular vector comprising a replication sequence, a first DNA sequence encoding the amino-terminal portion of the hybrid polypeptide, a second DNA sequence encoding the carboxy-terminal portion of said hybrid polypeptide. The circular vector is transformed into a rec positive microorganism in which the circular vector is amplified. This results in recombination of said circular vector mediated by the naturally occurring recombination mechanism of the rec positive microorganism, which include prokaryotes such as *Bacillus* and *E. coli,* and eukaryotes such as *Saccharomyces cerevisiae.*

Despite the existence of the above methods there are still need for even better iterative *in vivo* recombination methods for preparing Despite the existence of the above methods there are still need for even better iterative *in vivo* recombination methods for preparing novel positive polypeptide variants.

### SUMMARY OF THE INVENTION

The object of the present invention is to provide an improved method for preparing positive polypeptide variants by an *in vivo* recombination method.

The inventor of the present invention have surprisingly found that such positive polypeptide variants may advantageously be prepared by shuffling different nucleotide sequences of homologous DNA sequences by *in vivo* recombination comprising the steps of
a) forming at least one circular plasmid comprising a DNA sequence encoding a polypeptide,
b) opening said circular plasmid(s) within the DNA sequence(s) encoding the polypeptide(s),
c) preparing at least one DNA fragment comprising a DNA sequence homologous to at least a part of the polypeptide coding region on at least one of the circular plasmid(s), d) introducing at least one of said opened plasmid(s), together with at least one of said homologous DNA fragment(s) covering full-length DNA sequences encoding said polypeptide(s) or parts thereof, into a recombination host cell,
e) cultivating said recombination host cell, and
f) screening for positive polypeptide variants, wherein two or more opened plasmids are shuffled with one or more homologous DNA fragments in the same shuffling cycle.

### BRIEF DESCRIPTION OF THE DRAWINGS

Figure 1 shows the yeast expression plasmid pJSO26 comprising DNA sequence encoding the *Humicola lanuginosa* lipase gene.
Figure 2 shows the yeast expression plasmid pJSO37, comprising DNA sequence encoding the *Humicola lanuginosa* lipase gene containing twelve additional restriction sites.
Figure 3 shows the plasmid pJSO26.
Figure 4 shows the plasmid pJSO37.

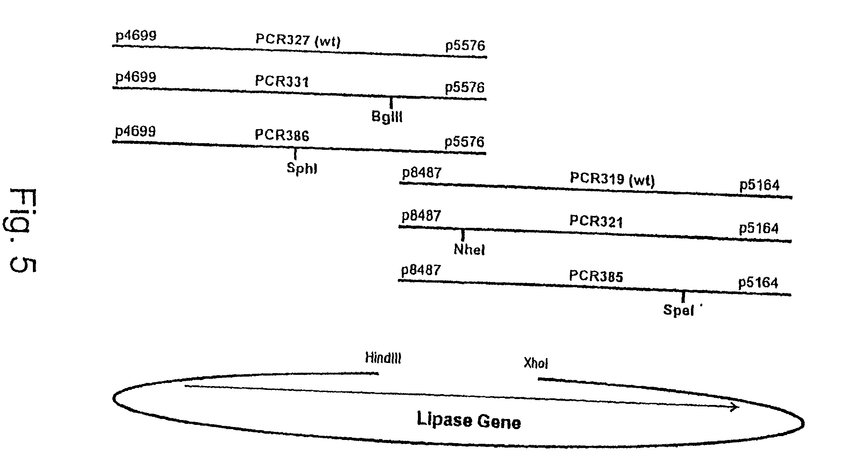

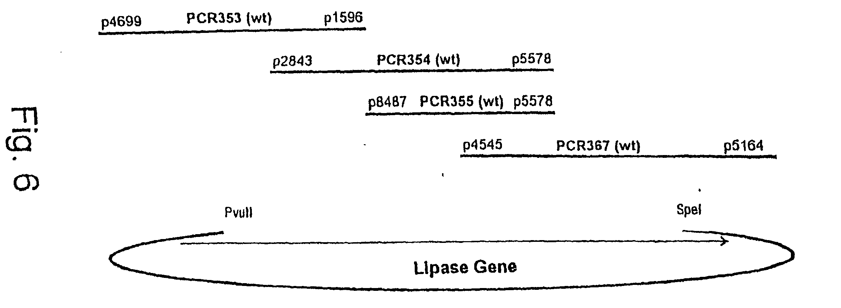

Figure 5 shows the *in vivo* recombination of the 0.9 kb synthetic wild-type *Humicola lanuginosa* lipase with pJSO37 using *Saccharomyces*

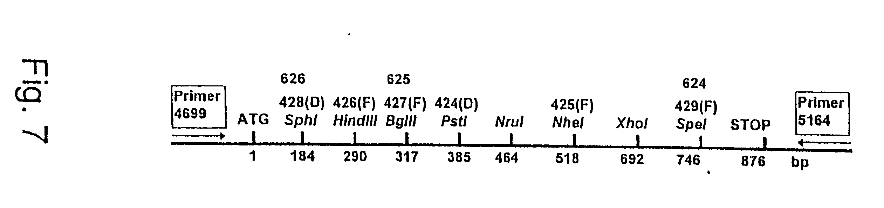

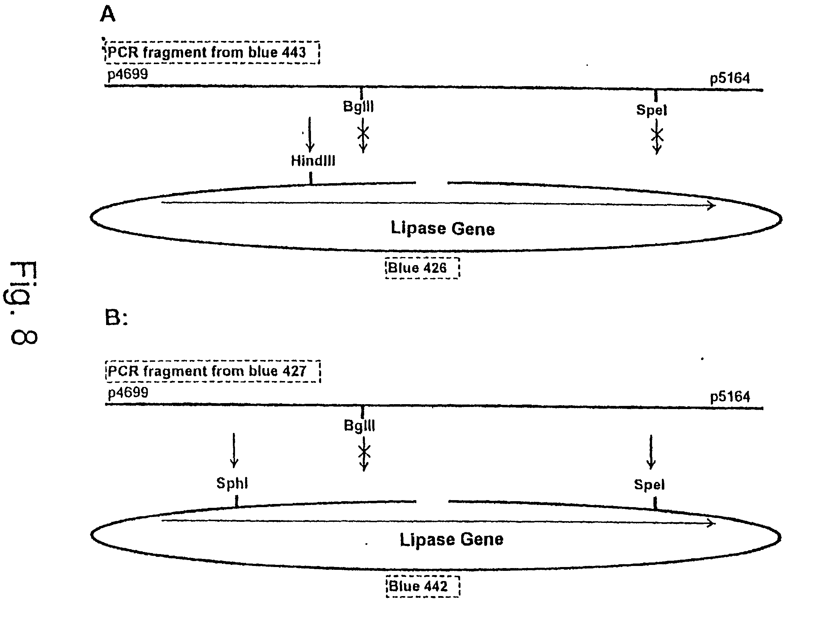

Figure 6 shows the *in vivo* recombination of a DNA fragment prepared from *Humicola lanuginosa* lipase variant (y) with *Humicola lanuginosa* lipase variant (d) comprised in a plasmid using *Saccharomyces cerevisiae* as the recombination host cell (described in Example 2).

### DETAILED DESCRIPTION OF THE INVENTION

The object of the present invention is to provide an improved method for preparing positive polypeptide variants by an iterative in *vivo* recombination method.

The inventor of the present invention have surprisingly found an efficient method for shuffling homologous DNA sequences in an *in vivo* recombination system using a eukaryotic cell as a recombination host cell.

A "recombination host cell" is in the context of the present invention a cell capable of mediating shuffling of a number of homologous DNA sequences.

The term "shuffling" means recombination of nucleotide sequence(s) between two or more homologous DNA sequences resulting in output DNA sequences (*i.e.* DNA sequences having been subjected to a shuffling cycle) having a number of nucleotides exchanged, in comparison to the input DNA sequences (*i.e.* starting point homologous DNA sequences).

An important advantage of the invention is that mosaic DNA sequences with multiple replacement points or replacements, not related to the opening site, is created, which is not discovered in Pompon's method.

An other important advantage of the present invention is that when using a mixture of fragments and opened vectors (in the screening set up) it gives the possibility of many different clones to recombine pairwise or even triplewise (as can be seen in a couple of examples below).

The *in vivo* recombination method of the invention simple to perform and results in a high level of mixing of homologous genes or variants. A large number of variants or homologous genes can be mixed in one transformation. The mixing of improved variants or wild type genes followed by screening increases the number of further improved variants manyfold compared to doing only random mutagenesis.

Recombination of multiple overlapping fragments is possible with a high efficiency increasing the mixing of variants or homologous genes using the in vivo recombination method. An overlap as small as 10 bp is sufficient for recombination which may be utilized for very easy domain shuffling of even distantly related genes.

The invention relates to a method for preparing polypeptide variants by shuffling different nucleotide sequences of homologous DNA sequences by *in vivo* recombination comprising the steps of
a) forming at least one circular plasmid comprising a DNA sequence encoding a polypeptide,
b) opening said circular plasmid(s) within the DNA sequence (s)

a) forming at least one circular plasmid comprising a DNA sequence encoding a polypeptide,
b) opening said circular plasmid(s) within the DNA sequence(s) encoding the polypeptide(s),
c) preparing at least one DNA fragment comprising a DNA sequence homologous to at least a part of the polypeptide coding region on at least one of the circular plasmid(s), d) introducing at least one of said opened plasmid(s), together with at least one of said homologous DNA fragment(s) covering full-length DNA sequences encoding said polypeptide(s) or parts thereof, into a recombination host cell,
e) cultivating said recombination host cell, and
f) screening for positive polypeptide variants, wherein two or more opened plasmids are shuffled with one or more homologous DNA fragments in the same shuffling cycle.

According to the invention more than one cycle of step a) to f) may be performed.

The opening of the plasmid(s) in step b) can be directed toward any site within the polypeptide coding region of the plasmid. The plamid(s) may be opened by any suitable methods known in the art. The opened ends of the plasmid may be filled-in with nucleotides as described in Pompon et al. (1989), supra). It is preferred not to fill in the opened ends as it might create a frameshift.

It is preferred to open the plasmid(s) around the middle of the polypeptide coding DNA sequence(s), as this is believed to result in a more effective recombination between DNA fragment(s) and opened plasmid(s).

In an embodiment of the invention the DNA fragment(s) is(are) prepared under conditions resulting in a low, medium or high random mutagenesis frequency.

To obtain low mutagenesis frequency the DNA sequence(s) (comprising the DNA fragment(s)) may be prepared by a standard PCR amplification method (US 4,683,202 or Saiki et al., (1988), Science 239, 487 - 491).

A medium or high mutagenesis frequency may be obtained by performing the PCR amplification under conditions which increase the mis-incorporation of nucleotides, for instance as described by Deshler. No. 1, 11-15.

It is also contemplated according to the invention to combine the PCR amplification (*i.e.* according to this embodiment also DNA fragment mutation) with a mutagenesis step using a suitable physical or chemical mutagenizing agent, *e.g.*, one which induces transitions, transversions, inversions, scrambling, deletions, and/or insertions.

In the context of the present invention the term "positive polypeptide variants" means resulting polypeptide variants possessing functional properties which has been improved in comparison to the polypeptides producible from the corresponding input DNA sequences. Examples, of such improved properties can be as different as *e.g*. biological activity, enzyme washing performance, antibiotic resistance etc.

Consequently, which screening method to be used for identifying positive variants depend on the desired improved property of the polypeptide variant in question.

If, for instance, the polypeptide in question is an enzyme and the desired improved functional property is the wash performance, the screening in step f) may conveniently be performed by use of a filter assay based on the following principle:
The recombination host cell is incubated on a suitable medium and under suitable conditions for the enzyme to be secreted, the medium being provided with a double filter comprising a first protein-binding filter and on top of that a second filter exhibiting a low protein binding capability. The recombination host cell is located on the second filter. Subsequent to the incubation, the first filter comprising the enzyme secreted from the recombination host cell is separated from the second filter comprising said cells. The first filter is subjected to screening for the desired enzymatic activity and the corresponding microbial colonies present on the second filter are identified.
The filter used for binding the enzymatic activity may be any protein binding filter *e.g.* nylon or nitrocellulose. The topfilter carrying the colonies of the expression organism may be any filter that has no or low affinity for binding proteins *e.g.* cellulose acetate or DuraporeÔ. The filter may be pre-treated with any of the conditions to be used for screening or may be treated during the detection of enzymatic activity.

The enzymatic activity may be detected by a dye, fluorescence, precipitation, pH indicator, IR-absorbance or any other known technique for detection of enzymatic activity.

The detecting compound may be immobilized by any immobilizing agent *e.g.* agarose, agar, gelatine, polyacrylamide, starch, filter paper, cloth; or any combination of immobilizing agents.

If the improved functional property of the polypeptide is not sufficiently good after one cycle of shuffling, the polypeptide may be subjected to another cycle.

In an embodiment of the invention at least one shuffling cycle is a backcrossing cycle with the initially used DNA fragment, which may be the wild-type DNA fragment. This eliminates non-essential mutations. Non-essential mutations may also be eliminated by using wild-type DNA fragments as the initially used input DNA material.

It is to be understood that the method of the invention is suitable for all types of polypeptide, including enzymes such as proteases, amylases, lipases, cutinases, amylases, cellulases, peroxidases and oxidases.

Also contemplated according to the invention is polypeptides having biological activity such as insulin, ACTH, glucagon, somatostatin, somatotropin, thymosin, parathyroid hormone, pigmentary hormones, somatomedin, erythropoietin, luteinizing hormone, chorionic gonadotropin, hypothalamic releasing factors, antidiuretic hormones, thyroid stimulating hormone, relaxin, interferon, thrombopoietin (TPO) and prolactin.

Especially contemplated according to the present invention is initially to use input DNA sequences being either wild-type, variant or modified DNA sequences, such as a DNA sequences coding for wild-type, variant or modified enzymes, respectively, in particular enzymes exhibiting lipolytic activity.

In an embodiment of the invention the lipolytic activity is a lipase activity derived from the filamentous fungi of the *Humicola sp.,* in particular *Humicola lanuginosa,* especially *Humicola lanuginosa.*

In a specific embodiment of the invention the initially used input DNA fragment to be shuffled with a homologous polypeptide is the wild-type DNA sequence encoding the *Humicola lanuginosa* lipase derived from *Humicola lanuginosa* DSM 4109 described in EP 305 216 (Novo Nordisk A/S).

Also specifically encompassed by the scope of the invention is input DNA sequences selected from the group of vectors (a) to (f) and/or DNA fragments (g) to (aa) coding for *Humicola lanuginosa* lipase variants from the list below in the Material and Method section.

Throughout the present application the name *Humicola lanuginosa* has been used to identify one preferred parent enzyme, *i.e.* the one mentioned immediately above. However, in recent years H. *lanuginosa* has also been termed *Thelmouyces lanuginosus* (a species introduced the first time by Tsiklinsky in 1989) since the fungus show morphological and physiological similarity to *Thermomyces lanuginosus.* Accordingly, it will be understood that whenever reference is made to *H. lanuginosa* this term could be replaced by *Thermomyces lanuginosus.* The DNA encoding part of the 18S ribosomal gene from *Thermomyces lanuginosus (or H. lanuginosa)* have been sequenced. The resulting 18S sequence was compared to other 18S sequences in the GenBank database and a phylogenetic analysis using parsimony (PAUP, Version 3.1.1, Smithsonian Institution, 1993) have also been made. This clearly assigns *Thermomyces lanuginosus* to the class of *Plectomycetes,* probably to the order of *Eurotiales.* According to the Entrez Browser at the NCBI (National Center for Biotechnology Information), this relates *Thermomyces lanuginosus* to families like *Eremascaceae, Monoascaceae, Pseudoeurotiaceae* and *Trichocomaceae,* the latter containing genera like *Emericella, Aspergillus, Penicillium, Eupenicillium, Paecilomyces, Talaromyces, Thermoascus* and *Sclerocleista.*

Consequently, such genes encoding lipolytic enzymes of filamentous fungi of the genera *Emericella, Aspergillus, Penicillium, Eupenicillium, Paecilomyces, Talaromyces, Thermoascus* and *Sclerocleista* are also specifically contemplated according to the present invention.

Other examples of relevant filamentous fungi genes encoding lipolytic enzymes include strains of the Absidia sp. *e.g.* the strains listed in WO 96/13578 (from Novo Nordisk A/S) which are hereby incorporated by reference. *Absidia* sp. strains listed in WO 96/13578 include *Absidia blakesleeana, Absidia corymbifera and Absidia reflexa.*

Strains of *Rhizopus* sp., in particular *Rh. niveus and* Rh. oryzea are also contemplated according to the invention.

The lipolytic gene may also be derived from a bacteria, such as a strain of the *Pseudomonas* sp., in particular *Ps. fragi, Ps. stutzeri, Ps. cepacia* and *Ps. fluorescens* (WO 89/04361), or *Ps. plantarii* or *Ps. gladioli* (US 4,950,417) or *Ps. alcaligenes* and *Ps. pseudoalcaligenes* (EP 218 272, EP 331 376, or WO 94/25578 (disclosing variants of the *Ps. pseudoalcaligenes* lipolytic enzyme), the *Pseudomonas* sp. variants disclosed in EP 407 225, or a *Pseudomonas* sp. lipolytic enzyme, such as the *Ps. mendocina* (also termed *Ps. putida*) lipolytic enzyme described in WO 88/09367 and US 5,389,536 or variants thereof as described in US 5,352,594, or *Ps. auroginosa or Ps. glumae, or Ps. syringae,* or *Ps. wisconsinensis (WO 96*/*12012 from Solvay) or a* strain of *Bacillus sp., e.g.* the *B. subtilis* described by Dartois et al., (1993) Biochemica et Biophysica acta 1131, 253-260, or *B. stearothermophilus (JP* 64/7744992) or *B. pumilus* (WO 91/16422) or a strain of *Streptomyces* sp., *e.g. S. scabies,* or a strain of *Chromobacterium* sp. *e.g C. viscosum.*

In connection with the *Pseudomonas sp.* lipases it has been found that lipases from the following organisms have a high degree of homology, such as at least 60% homology, at least 80% homology or at least 90% homology, and thus are contemplated to belong to the same family of lipases: *Ps.* ATCC21808, *Pseudomonas* sp. lipase commercially available as Liposam© , Ps. *aeruginosa* EF2, *Ps. aeruginosa* PAC1R, *Ps. aeruginosa* PAO1, Ps. *aeruginosa* TE 3285, *Ps. sp.* 109, *Ps. pseudoalcaligenes* M1, *Ps. glumae, Ps. cepacia* DSM 3959, *Ps. cepacia* M-12-33, *Ps. sp.* KWI-56, *Ps. putida* IFO 3458, *Ps. putida* IFO 12049 (Gilbert, E. J., (1993), Pseudomonas lipases: Biochemical properties and molecular cloning. Enzyme Microb. Technol., 15, 634-645). The species *Pseudomonas cepacia* has recently been reclassified as *Burkholderia cepacia,* but is termed *Ps. cepacia* in the present application.

Also genes encoding lipolytic enzymes from yeasts are relevant, ans include lipolytic genes from *Candida* sp., in particular *Candida rugosa,* or *Geotrichum sp., in particular Geotrichum candidum.*

Specific examples of microorganisms comprising genes encoding lipolytic enzymes used for commercially available products and which may serve as donor of genes to be shuffled according to the invention include *Humicola lanuginosa,* used in Lipolase®, Lipolase® Ultra, *Ps. mendocina* used in Lumafast®, *Ps. alcaligenes* used in Lipomax®, *Fusarium solani, Bacillus* sp. (US 5427936, EP 528828), *Ps. mendocina,* used in Liposam® .

It is to be emphasized that genes encoding lipolytic enzyme to be shuffled according to the invention may be any of the above mentioned genes of lipolytic enzymes and any variant, modification, or truncation thereof. Examples of such genes which are specifically contemplated include the genes encoding the enzymes described in WO 92/05249, WO 94/01541, WO 94/14951, WO 94/25577, WO 95/22615 and a protein engineered lipase variants as described in EP 407 225; a protein engineered *Ps. mendocina* lipase as described in US 5,352,594; a cutinase variant as described in WO 94/14964; a variant of an *Aspergillus* lipolytic enzyme as described in EP patent 167,309; and *Pseudomonas sp.* lipase described in WO 95/06720.

A request to the DNA sequences, encoding the polypeptide(s), to be shuffled, is that they are at least 60%, preferably at least 70%, better more than 80%, especially more than 90%, and even better up to almost 100% homologous. DNA sequences being less homologous will have less inclination to interact and recombine.

Also the *Pseudomonas* sp. lipase gene shown in SEQ ID NO. 14 are specifically contemplated according to the invention.

It is also contemplated according to the invention to shuffle parent (homologous) wildt type organisms of different genera.

Further, the DNA fragment(s) to be shuffled may preferably have a length of from about 20 bp to 8 kb, preferably about 40 bp to 6 kb, more preferred about 80 bp to 4 kb, especially about 100 bp to 2 kb, to be able to interact optimally with the opened plasmid.

The method of the invention is very efficient for preparing polypeptide variants in comparison to prior art method comprising transforming linear DNA fragments/sequences.

The inventor found that the transformation frequency of a mixture of opened plasmid and a DNA fragment were significantly higher than when transforming a plasmid cut at the same site alone. The transformation frequency of the opened plasmid and DNA fragment were as high as for uncut plasmid.

Without being limited to any theory it is believed that the opening of the plasmid(s) restrict(s) the replication of (opened) plasmid(s) when not interacting with at least one DNA fragment. In accordance with this an increased number of recombined DNA sequences were found after only one shuffling cycle.

As described in Example 1 50% of the resulting transformants contained recombined DNA sequences of both input DNA sequences. As high as 20% of the total number of recombined DNA sequences were "random" mixtures (*i.e.* having more than one region of nucleotides exchanged).

The input DNA sequences may be any DNA sequences including wild-type DNA sequences, DNA sequences encoding variants or mutants, or modifications thereof, such as extended or elongated DNA sequences, and may also be the outcome of DNA sequences having been subjected to one or more cycles of shuffling (*i.e.* output DNA sequences) according to the method of the invention or any other method (*e.g.* any of the methods described in the prior art section).

When using the method of the invention the output DNA sequences (*i.e.* shuffled DNA sequences), have had a number of nucleotide(s) exchanged. This results in replacement of at least one amino acid within the polypeptide variant, if comparing it with the parent polypeptide. It is to be understood that also silent mutations is contemplated (*i.e* nucleotide exchange which does not result in changes in the amino acid sequence).

However, the method of the present invention will in most cases lead to the replacement of a considerable number of amino acid and may in certain cases even alter the structure of one or more polypeptide domains (*i.e.* a folded unit of polypeptide structure).

According to the present invention more than two DNA sequences are shuffled at the same time. Actually any number of different DNA fragments and homologous polypeptides comprised in suitable plasmids may be shuffles at the same time. This is advantageous as a vast number of quite different variants can be made rapidly without an abundance of iterative procedures.

The inventor have tested the nucleotide shuffling method of the invention using significantly more than two homologous DNA sequences. As described in Example 2 it was surprisingly found that the method of the invention advantageously can be used for recombining more than two DNA sequences.

One cycle of shuffling according to the method of the invention may result in the exchange of from 1 to 1000 nucleotides into the opened plasmid DNA sequence encoding the polypeptide in question. The exchanged nucleotide sequence(s) may be continuous or may be present as a number of sub-sequences within the full-length sequence(s).

To support the present invention the inventor made a number of additional experiments on different aspect on the method of the invention. The experiments are described below and illustrated in the Example 3 to 6 below.

A number of vectors and fragments comprising an inactivated synthetic *Humicola lanuginosa* lipase genes were constructed by introducing frameshift/stop codon mutations in the lipase gene at various positions. These were used for monitoring the *in vivo* recombination of different combinations of opened vector(s) and DNA fragments. The number of active lipase colonies were scored as described in Example 3. The number of colonies determines the efficiency of the opened vector(s) and fragment(s) recombination.

One frameshift mutation in said *Humicola lanuginosa* lipase gene in the opened vector and another in the fragment on the opposite side of the opening site gave 3 to 32% of active lipase colonies depending on the location and combination. It was concluded that the closer that the mutation is at the ends of the vector the higher mixing.

One frameshift mutation in the opened vector and two in the fragment on each side of the opening site gave 4 to 42% of active colonies depending on the location and combination. Some of these active colonies can be considered to be mosaics, not only related to the opening site.

Two frameshift mutations in the opened vector on each side of the opening site and one in the fragment gave 0.5 to 3.1% of active colonies depending on the location and combination. Most of these active colonies are mosaics of the "parent" DNA.

Two frameshift mutations in the opened vector on each side of the opening site and a wild type fragment gave 7.7 to 10.7% of active colonies depending on the location.

It was also found that the amount of vectors relative to fragments and the size of the fragments are also influencing the result.

Using of the S. *cerevisiae* rad52 mutants as the recombination host cell showed that the *rad52* mutant transformed very well with wild type plasmid(s) and expressed the *Humicola lanuginosa* lipase gene, but gave no transformants at all with the opened vectors and fragments.

The RAD52 function is required for "classical recombination" (but not for unequal sister-strand mitotic recombination) showing that the recombination of opened vector and fragment could involve a classical recombination mechanism.

Classical recombination is the recombination mechanism involved in the recombination between genes located on nonsister chromatids of homologous chromosomes as defined in for example Petes TD, Malone RE and Symington LS (1991) "Recombination in Yeast", page 407-522, in The Molecular and Cellular Biology of the Yeast Saccharomyces, Volume 1 (eds. Broach JR, Pringle JR and Jones EW), Cold Spring Harbor Laboratory Press, New York.

### Multiple partially overlapping fragements

The inventor also tested recombination of multiple partial overlapping fragments using the method of the invention.

The recombination of 2 and 3 partial overlapping fragments into a gapped (*i.e.* that the opening result in cutting out of a little part of the gene) vector were tested and gave a high recovery of recombined *Humicola lanuginosa* lipase gene. The recovery of active lipase gene from different combinations of inactivated *Humicola lanuginosa* genes was tested for the recombination of 2 partial overlapping fragments. The tendency was a higher mixing in the overlapping region between the 2 fragments in the gapped region than in the vector and fragment overlap.

When recombining many fragments from the same region, the multiple overlapping fragment technique will increase the mixing by itself, but it is also important to have a relative high random mixing in overlapping regions in order to mix closely located variants/differences.

An overlap as small as 10 bp between two fragments were found to be sufficient to obtain a very efficient recombination. Therefore, overlapping in the range from 5 to 5000 bp, preferably from 10 bp to 500 bp, especially 10 bp to 100 bp is suitable according to the method of the invention.

According to this embodiment of the present invention 2 or more overlapping fragments, preferable 2 to 6 overlapping fragments, especially 2 to 4 overlapping fragments may advantageously be used as input fragments in a shuffling cycle.

Besides increasing the mixing of genes, this is a very useful method for domain shuffling by creating small overlaps between DNA fragments from different domains and screen for the best combination.

For instance, in the case of three DNA fragments the overlapping regions may be as follows:
- the first end of the first fragment overlaps the first end of the opened plasmid,
- the first end of the second fragment overlaps the second end of the first fragment, and the second end of the second fragment overlaps the first end of the third fragment,
- the first end of the third fragment overlaps (as stated above) the second end of the second fragment, and the second end of the third fragment overlaps the second end of the opened plasmid.

It is to be understood that when using two or more DNA fragments as starting material it is preferred to have continuos overlaps between the ends of the plasmid and the DNA fragments.

Even though it is preferred to shuffle homologous DNA sequences in the form of DNA fragment(s) and opened plasmid(s), it is also contemplated according to the invention to shuffle two or more opened plasmids comprising homologous DNA sequences encoding polypeptides. However, in such case it is compulsory to open the plasmids at different sites.

In an further embodiment of the invention two or more opened plasmids and one or more homologous DNA fragments are used as the starting material to be shuffled. The ratio between the opened plasmid(s) and homologous DNA fragment(s) preferably lie in the range from 20:1 to 1:50, preferable from 2:1 to 1:10 (mol vector:mol fragments) with the specific concentrations being from 1 pM to 10 M of the DNA.

The opened plasmids may advantagously be gapped in such a way that the overlap between the fragments is deleted in the vector in order to select for the recombination).

### Preparing the DNA fragment

The DNA fragment to be shuffled with the homologous polypeptide comprised in an opened plasmid may be prepared by any suitable method. For instance, the DNA fragment may be prepared by PCR amplification (polymerase chain reaction), as described above, of a plasmid or vector comprising the gene of the polypeptide, using specific primers, for instance as described in US 4,683,202 or Saiki et al., (1988), Science 239, 487 - 491. The DNA fragment may also be cut out from a vector or plasmid comprising the desired DNA sequence by digestion with restriction enzymes, followed by isolation using *e.g.* electrophoresis.

The DNA fragment encoding the homologous polypeptide in question may alternatively be prepared synthetically by established standard methods, *e.g.* the phosphoamidite method described by Beaucage and Caruthers, (1981), Tetrahedron Letters 22, 1859 - 1869, or the method described by Matthes et al., (1984), EMBO Journal 3, 801 - 805. According to the phosphoamidite method, oligonucleotides are synthesized, *e.g.* in an automatic DNA synthesizer, purified, annealed, ligated and cloned in suitable vectors.

Furthermore, the DNA fragment may be of mixed synthetic and genomic, mixed synthetic and cDNA or mixed genomic and cDNA origin prepared by ligating fragments of synthetic, genomic or cDNA origin (as appropriate), the fragments corresponding to various parts of the entire DNA sequence, in accordance with standard techniques.

### The plasmid

The plasmid comprising the DNA sequence encoding the polypeptide in question may be prepared by ligating said DNA sequence into a suitable vector or plasmid, or by any other suitable method.

Said vector may be any vector which may conveniently be subjected to recombinant DNA procedures. The choice of vector will often depend on the recombination host cell into which it is to be introduced.

Thus, the vector may be an autonomously replicating vector, i.e. a vector which exists as an extrachromosomal entity, the replication of which is independent of chromosomal replication, *e.g.* a plasmid. Alternatively, the vector may be one which, when introduced into the recombination host cell, is integrated into the host cell genome and replicated together with the chromosome(s) into which it has been integrated.

To facilitate the screening process it is preferred that the vector is an expression vector in which the DNA sequence encoding the polypeptide in question is operably linked to additional segments required for transcription of the DNA. In general, the expression vector is derived from a plasmid, a cosmid or a bacteriophage, or may contain elements of any or all of these.

The term, "operably linked" indicates that the segments are arranged so that they function in concert for their intended purposes, *e.g.* transcription initiates in a promoter and proceeds through the DNA sequence coding for the polypeptide in question.

The promoter may be any DNA sequence which shows transcriptional activity in the recombination host cell of choice and may be derived from genes encoding proteins, such as enzymes, either homologous or heterologous to the host cell.

Examples of suitable promoters for use in yeast host cells include promoters from yeast glycolytic genes (Hitzeman et al.,(1980), J. Biol. Chem. 255, 12073 - 12080; Alber and Kawasaki, (1982), J. Mol. Appl. Gen. 1, 419 - 434) or alcohol dehydrogenase genes (Young et al., in Genetic Engineering of Microorganisms for Chemicals (Hollaender et al, eds.), Plenum Press, New York, 1982), or the TPI1 (US 4,599,311) or ADH2-4c (Russell et al., (1983), Nature 304, 652 - 654) promoters.

Examples of suitable promoters for use in filamentous fungus host cells are, for instance, the ADH3 promoter (McKnight et al., (1985), The EMBO J. 4, 2093 - 2099) or the tpiA promoter. Examples of other useful promoters are those derived from the gene encoding A. *oryzae* TAKA amylase, *Rhizomucor miehei* aspartic proteinase, A. *niger* neutral a-amylase, *A. niger* acid stable a-amylase, *A. niger* or *A. awamori* glucoamylase (gluA), *Rhizomucor miehei* lipase, *A. oryzae* alkaline protease, *A. oryzae* triose phosphate isomerase or *A. nidulans* acetamidase. Preferred are the TAKA-amylase and gluA promoters.

The DNA sequence encoding polypeptide in question invention may also, if necessary, be operably connected to a suitable terminator, such as the human growth hormone terminator (Palmiter et al., op. cit.) or (for fungal hosts) the TPI1 (Alber and Kawasaki, op. cit.) or ADH3 (McKnight et al., op. cit.) terminators. The vector may further comprise elements such as polyadenylation signals (*e.g.* from SV40 or the adenovirus 5 Elb region), transcriptional enhancer sequences (*e.g.* the SV40 enhancer) and translational enhancer sequences (*e.g.* the ones encoding adenovirus VA RNAs).

The vector may further comprise a DNA sequence enabling the vector to replicate in the recombination host cell in question. When the host cell is a yeast cell, suitable sequences enabling the vector to replicate are the yeast plasmid 2m replication genes REP 1-3 and origin of replication.

The plasmid pYl can be used for production of useful proteins and peptides, using filamentous fungi, such *as Aspergillus* sp., and yeasts as recombinant host cells (JP06245777-A).

The vector may also comprise a selectable marker, *e.g.* a gene the product of which complements a defect in the recombination host cell, such as the gene coding for dihydrofolate reductase (DHFR) or the *Schizosaccharomyces pombe* TPI gene (described by P.R. Russell, (1985), Gene 40, 125-130).

Another example of such suitable selective markers are the ura3 and leu2 genes which complements the corresponding defect genes of *e.g.* the yeast strain *Saccharomyces cerevisiae* YNG318.

The vector may also comprise a selectable marker which confers resistance to a drug, *e.g*. ampicillin, kanamycin, tetracyclin, chloramphenicol, neomycin, hygromycin or methotrexate. For filamentous fungi, selectable markers include amdS, pyrG, argB, niaD, sC, trpC, pyr4, and DHFR.

To direct the polypeptide in question into the secretory pathway of the recombination host cell, a secretory signal sequence (also known as a leader sequence, prepro sequence or pre sequence) may be provided in the recombinant vector. The secretory signal sequence is joined to the DNA sequence encoding the lipolytic enzyme in the correct reading frame. Secretory signal sequences are commonly positioned 5' to the DNA sequence encoding the polypeptide. The secretory signal sequence may be the signal normally associated with the polypeptide in question or may be from a gene encoding another secreted protein.

The signal peptide may be naturally occurring signal peptide, or a functional part thereof, or it may be a synthetic peptide. For secretion from yeast cells, suitable signal peptides have been found to be the a-factor signal peptide (cf. US 4,870,008), the signal peptide of mouse salivary amylase · (cf. O. Hagenbuchle et al., (1981), Nature 289, 643-646), a modified carboxypeptidase signal peptide (cf. L.A. Valls et al., (1987), Cell 48, 887-897), the *Humicola lanuginosa* lipase signal peptide, the yeast BAR1 signal peptide (cf. WO 87/02670), or the yeast aspartic protease 3 (YAP3) signal peptide (cf. M. Egel-Mitani et al., (1990), Yeast 6, 127-137).

For efficient secretion in yeast, a sequence encoding a leader peptide may also be inserted downstream of the signal sequence and upstream of the DNA sequence encoding the polypeptide in question. The function of the leader peptide is to allow the expressed polypeptide to be directed from the endoplasmic reticulum to the Golgi apparatus and further to a secretory vesicle for secretion into the culture medium (*i.e.* exportation of the polypeptide across the cell wall or at least through the cellular membrane into the periplasmic space of the yeast cell). The leader peptide may be the yeast a-factor leader (the use of which is described in *e.g.* US 4,546,082, EP 16 201, EP 123 294, EP 123 544 and EP 163 529). Alternatively, the leader peptide may be a synthetic leader peptide, which is to say a leader peptide not found in nature. Synthetic leader peptides may, for instance, be constructed as described in WO 89/02463 or WO 92/11378.

For use in filamentous fungi, the signal peptide may conveniently be derived from a gene encoding an *Aspergillus* sp. amylase or glucoamylase, a gene encoding a *Rhizomucor miehei* lipase or protease, a *Humicola lanuginosa* lipase. The signal peptide is preferably derived from a gene encoding *A. oryzae* TAKA amylase, *A. niger* neutral α-amylase, *A. niger* acid-stable amylase, or A. *niger* glucoamylase.

### The recombination host cell

The recombination host cell, into which the mixture of plasmid/fragment DNA sequences are to be introduced, may be any eukaryotic cell, including fungal cells and plant cells, capable of recombining the homologous DNA sequences in question.

According to prior art prokaryotic microorganisms, such as bacteria including *Bacillus* and *E. coli;* eukaryotic organisms, such as filamentous fungi, including *Aspergillus* and yeasts such as *Saccharomyces cerevisiae;* and tissue culture cells from avian or mammalian origins have been suggested for in *vivo* recombination. All of said organisms can be used as recombination host cell, but in general prokaryotic cells are not sufficiently effective *(i.e.* does not result in a sufficient number of variants) to be suitable for recombination methods for industrial use.

Consequently, preferred recombination host cells according to the present invention are fungal cells, such as yeast cells or filamentous fungi.

Examples of suitable yeast cells include cells of *Saccharomyces* sp., in particular strains of *Saccharomyces cerevisiae* or *Saccharomyces kluyveri* or *Schizosaccharomyces* sp., Methods for transforming yeast cells with heterologous DNA and producing heterologous polypeptides therefrom are described, *e.g.* in US 4,599,311, US 4,931,373, US 4,870,008, 5,037,743, and US 4,845,075, all of which are hereby incorporated by reference. Transformed cells may be selected by, *e.g.*, a phenotype determined by a selectable marker, commonly drug resistance or the ability to grow in the absence of a particular nutrient, *e.g.* leucine. A preferred vector for use in yeast is the POT1 vector disclosed in US 4,931,373. The DNA sequence encoding the polypeptide may be preceded by a signal sequence and optionally a leader sequence, *e.g.* as described above. Further examples of suitable yeast cells are strains of *Kluyveromyces*, such as K. *lactis, Hansenula, e.g. H. polymorpha, or Pichia, e.g. P. pastoris* (cf. Gleeson et al.,(1986), J. Gen. Microbiol. 132, 3459-3465; US 4,882,279).

Examples of other fungal cells are cells of filamentous fungi, *e.g. Aspergillus* sp*., Neurospora* sp., *Fusarium* sp. *or Trichoderma* sp., in particular strains of *A. oryzae, A. nidulans* or *A. niger.* The use of *Aspergillus* sp. for the expression of proteins is described in, *e.g.,* EP 272 277, EP 230 023. The transformation of *F. oxysporum* may, for instance, be carried out as described by Malardier et al., (1989), Gene 78, 147-156.

In a preferred embodiment of the invention the recombination host cell is a cell of the genus *Saccharomyces, in* particular *S*. *cerevisiae.*

### METHODS AND MATERIALS

### DNA sequence:

*Humicola lanuginosa* DSM 4109 derived lipase encoding DNA sequence.

### Humicola lanuginosa lipase variants:

### Variants used for preparing vectors to be opened with NruI in Example 2:

(a) E56R,D57L,I90F,D96L,E99K
(b) E56R,D57L,V60M,D62N,S83T,D96P,D102E
(c) D57G,N94K,D96L,L97M
(d) E87K,G91A,D96R,I100V,E129K,K237M,I252L,P256T,G263A,L264Q
(e) E56R,D57G,S58F,D62C,T64R,E87G,G91A,F95L,D96P,K98I,(K237M)
(f) E210K

### Variants used for preparing DNA fragments by standard PCR amplification in Example 2:

(g) S83T,N94K,D96N
(h) E87K,D96V
(i) N94K,D96A
(j) E87K,G91A,D96A
(k) D167G,E210V
(l) S83T,G91A,Q249R
(m) E87K,G91A
(n) S83T,E87K,G91A,N94K,D96N,D111N.
(o) N73D,E87K,G91A,N94I,D96G.
(p) L67P,I76V,S83T,E87N,I90N,G91A,D96A,K98R.
(q) S83T,E87K,G91A,N92H,N94K,D96M
(s) S85P,E87K,G91A,D96L,L97V.
(t) E87K,I90N,G91A,N94S,D96N,I100T.
(u) I34V,S54P,F80L,S85T,D96G,R108W,G109V,D111G,S116P,L124S, V132M,V140Q,V141A,F142S,H145R,N162T,I166V,F181P,F183S, R205G, A243T,D254G,F262L.
(v) E56R,D57L,I90F,D96L,E99K
(x) E56R,D57L,V60M,D62N, S83T,D96P,D102E
(y) D57G,N94K,D96L,L97M
(z) E87K,G91A,D96R,I100V,E129K,K237M,I252L,P256T,G263A,L264Q
(aa) E56R,D57G,S58F,D62C,T64R,E87G,G91A,F95L,D96P,K98I

### Strains:

### Expression system host:

*Saccharomyces cerevisiae* YNG318: MATa Dpep4[cir⁺] ura3-52, leu2-D2, his 4-539
*Saccharomyces cerevisiae Rad52: Strain M1533 = MATa rad52 ura3,* obtained from Torsten Nilsson Tillgren, Institute of Genetics, University of Copenhagen.

### Plasmids:

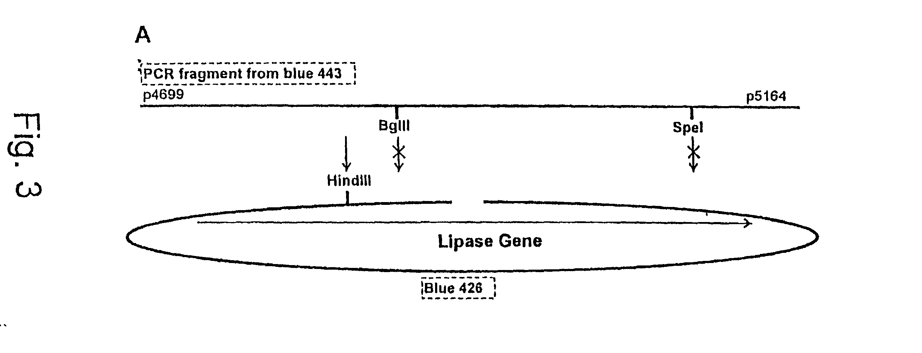

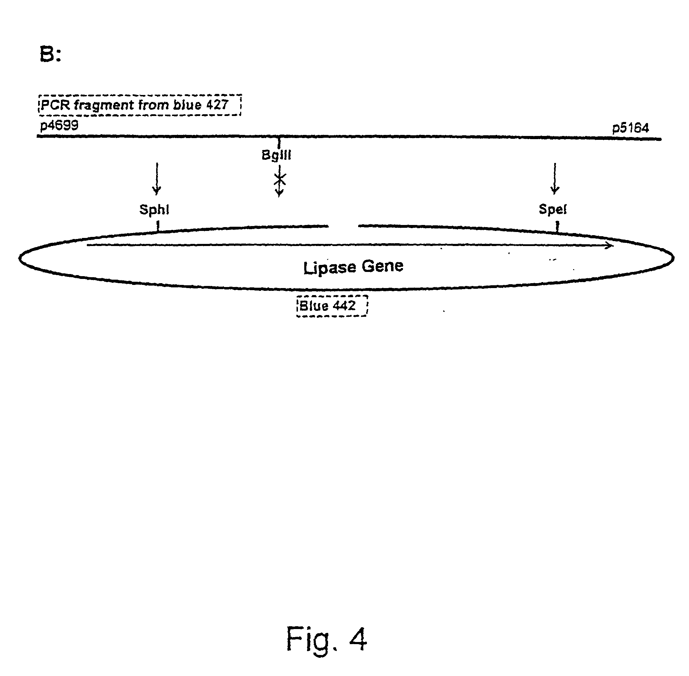

pJSO26 (see figure 3)
pJSO37 (see figure 4)
pYES 2.0 (Invitrogen)

### Transformation selective marker

ura3
leu2

### Media

SC-ura⁻: 90 ml 10 x Basal salt, 22.5 ml 20% casamino acids, 9 ml 1% tryptophan, H₂O ad 806 ml, autoclaved, 3.6 ml 5% threonine and 90 ml 20% glucose or 20% galactose added.
LB-medium: 10 g Bacto-tryptone, 5 g Bacto yeast extract, 10 g NaCl in 1 litre water.
Brilliant Green (BG) (Merck, art. No. 1.01310)
BG-reagent: 4 mg/ml Brilliant Green (BG) dissolved in water Substrate 1:
10 ml olive oil (Sigma CAT NO. 0-1500)
20 ml 2% polyvinyl alcohol (PVA)
The Substrate is homogenised for 15-20 minutes.

### Methods:

### Construction of yeast expression vector

The expression plasmids pJSO26 and pJSO37, are derived from pYES 2.0. The inducible GAL1-promoter of pYES 2.0 was replaced with the constitutively expressed TPI (triose phosphate isomerase)-promoter from *Saccharomyces cerevisiae* (Albert and Karwasaki, (1982), J. Mol. Appl Genet., 1, 419-434), and the ura3 promoter has been deleted. A restriction map of pJSO26 and pJSO37 is shown in figure 3 and figure 4, respectively.

### Preparation of the wild-type DNA fragment

A lipase wild-type DNA fragment can be prepared either by PCR amplification (resulting in low, medium or high mutagenesis), of the pJSO26 plasmid or by cutting the DNA fragment out by digesting with a suitable restriction enzyme.

### Fermentation of Humicola lanuginosa lipase variants in yeast

10 ml of SC-ura⁻ medium is inoculated with a *S*. *cerevisiae* colony and grown at 30°C for 2 days. The 10 ml is used for inoculating 300 ml SC-ura⁻ medium which is grown at 30°C for 3 days. The 300 ml is used for inoculation 5 1 of the following G-substrate:

| | |
|---|---|
| 400 g | Amicase |
| 6.7 g | yeast extract (Difco) |
| 12.5 g | L-Leucin (Fluka) |
| 6.7 g | (NH₄)₂SO₄ |
| 10 g | MgSO₄^{.}7H₂O |
| 17 g | K₂SO₄ |
| 10 ml | Trace compounds |
| 5 ml | Vitamin solution |
| 6.7 ml | H₃PO₄ |
| 25 ml | 20% Pluronic (antifoam) |

### In a total volume of 5000 ml:

The yeast cells are fermented for 5 days at 30°C. They are given a start dosage of 100 ml 70% glucose and added 400 ml 70% glucose/day. A pH=5.0 is kept by addition of a 10% NH₃ solution. Agitation is 300 rpm for the first 22 hours followed by 900 rpm for the rest of the fermentation. Air is given with 11 air/l/min for the first 22 hours followed by 1.5 l air/l/min for the rest of the fermentation.

| Trace compounds: | |
|---|---|
| 6.8 g | ZnCl₂ |
| 54.0 g | FeCl₂·6H₂O |
| 19.1 g | MnCl₂·4H₂O |
| 2.2 g | CuSO₄·5H₂O |
| 2.58 g | CoCl₂ |
| 0.62 g | H₃BO₃ |
| 0.024 g | (NH₄)₆Mo₇O₂₄·4H₂O |
| 0.2 g | KI |
| 100 ml | HCl (concentrated) |
| In a total volume of 1 l. | |

| Vitamin solution: | |
|---|---|
| 250 mg | Biotin |
| 3 g | Thiamin |
| 10 g | D-Calciumpanthetonat |
| 100 g | Myo-Inositol |
| 50 g | Cholinchlorid |
| 1.6 g | Pyridoxin |
| 1.2 g | Niacinamid |
| 0.4 g | Folicacid |
| 0.4 g | Riboflavin |
| In a total volume of 1l. | |

### Transformation of yeast

*Saccharomyces cerevisiae* is transformed by standard methods (cf. Sambrooks et al., (1989), Molecular Cloning: A Laboratory Manual, 2nd Ed., Cold Spring Harbor)

### Determination of yeast transformation frequency

The transformation frequency is determined by cultivating the transformants on SC-ura plates for 3 days and counting the number of colonies appearing. The number of transformants per mg opened plasmid is the transformation frequency.

### Screening for positive variants with improved wash performance

The following filter assay can be used for screening positive variants with improved wash performance.

Low calcium filter assay
1) Provide SC Ura⁻ replica plates (useful for selecting strains carrying the expression vector) with a first protein binding filter (Nylon membrane) and a second low protein binding filter (Cellulose acetate) on the top.
2) Spread yeast cells containing a parent lipase gene or a mutated lipase gene on the double filter and incubate for 2 or 3 days at 30°C.
3) Keep the colonies on the top filter by transferring the topfilter to a new plate.
4) Remove the protein binding filter to an empty petri dish.
5) Pour an agarose solution comprising an olive oil emulsion (2% PVA:olive oil=3:1), Brilliant green (indicator,0.004%), 100 mM tris buffer pH9 and EGTA (final concentration 5mM) on the bottom filter so as to identify colonies expressing lipase activity in the form of blue-green spots.
6) Identify colonies found in step 5) having a reduced dependency for calcium as compared to the parent lipase.

DNA sequencing was performed by using applied Biosystems ABI DNA sequence model 373A according to the protocol in the ABI Dye Terminator Cycle Sequencing kit.

### Assessing the effiency of recombination

The number of colonies determines the efficiency of the opened vector and fragment recombination. The percentage of colonies with active lipase activity gives an estimate of the mixing of the active and inactive genes - theoretically it can be calculated for one frameshift that the closer to 50% the better mixing if equal likelihood of. wild type and frameshift, 25% for 2 frameshifts and 12.5% for 3 frameshifts.

### Frameshift mutation

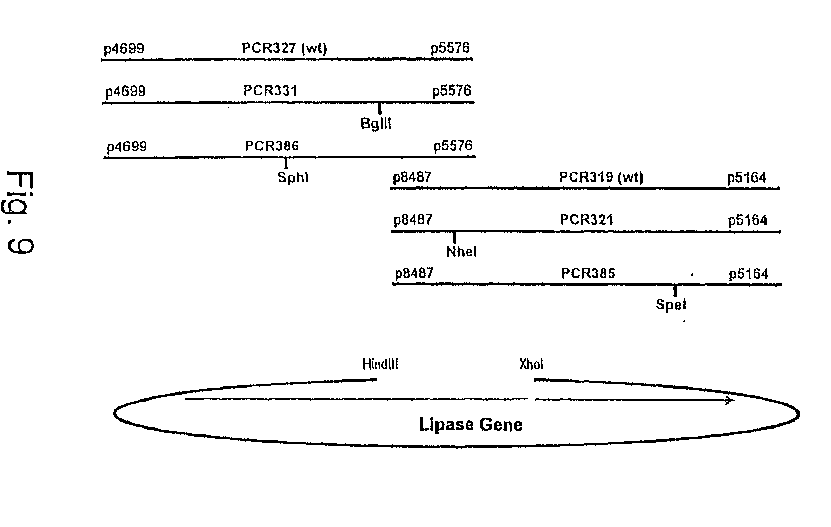

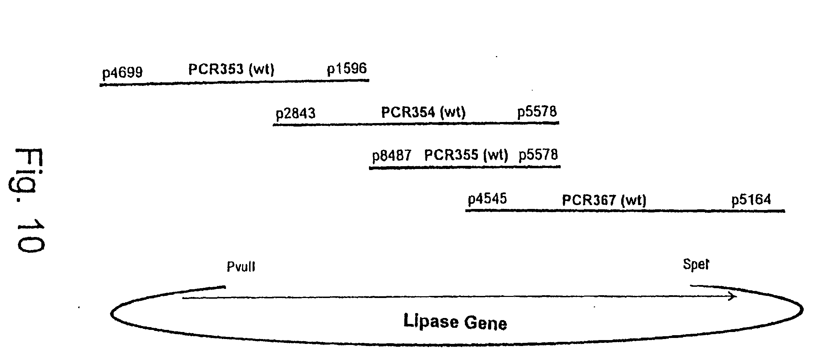

The frameshift mutation were created either by filling in a restriction site (in case of 5' overhang) or deleting the "sticky ends" (in case of 3' overhang) by T4 DNA polymerase with or without dNTP (deoxynucleotides = equal amounts of dATP, dTTP, dCTP and dGTP). Methods for filling in of restriction sites (referred to as "F" on Figure 7) and deleting the sticky ends (referred to as "(D)" on Figure 7) are well known in the art.

### Method for assessing colonies with lipase activity

The number of colonies and positives (*i.e.* with lipase activity) are calculated as the average of 3 plates. The cultivation condition and screening condition used is the following:
1) Provide SC Ura-plates with a protein binding filter (Nylon filter) onto the plate.
2) Spread yeast cells containing a parent lipase gene or a mutated lipase gene on the filter and incubate for 3 or 4 days at 30°C.
3) Remove the protein binding filter with the colonies to a petri dish containing: An agarose solution comprising an olive oil emulsion (2% PVA:Olive oil=2:1), Brilliant green (indicator,0.004%), 100 mM tris buffer pH 9.
5) Identify colonies expressing lipase activity in the form of blue-green spots.

### EXAMPLES

### Example 1

### Testing in vivo recombination of two homologous genes

The *Saccharamyces cerevisiae* expression plasmid pJSO26 was constructed as described above in the "Material and Methods"-section.

A synthetic *Humicola lanuginosa* lipase gene (in pJS037) containing 12 additional restriction sites (see figure 4) was cut with NruI, PstI, and NruI and PstI, respectively, to open the gene approximately in the middle of the DNA sequence encoding the lipase.

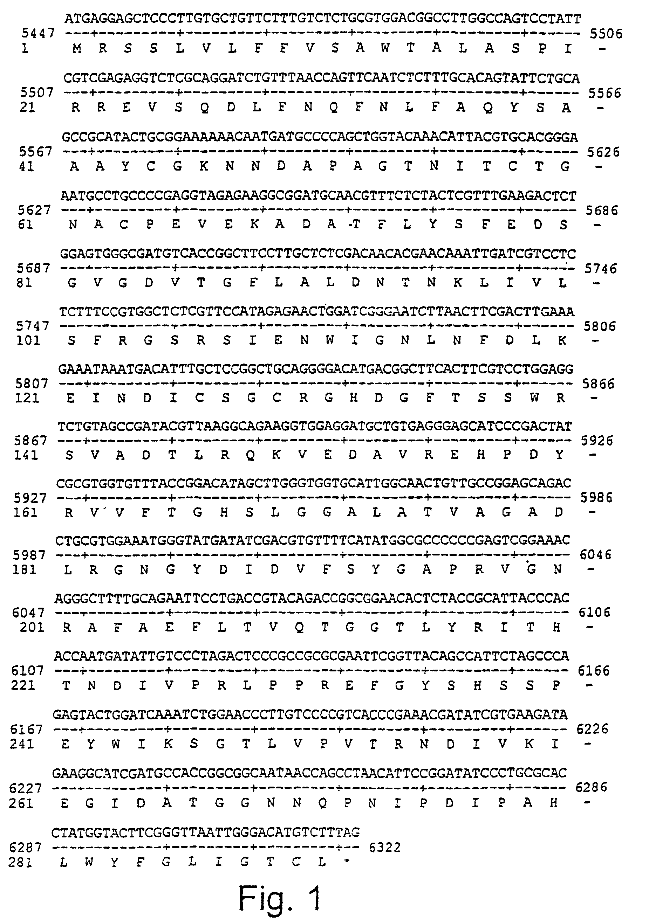

The opened plasmid (pJSO37) was transformed into *Saccharomyces cerevisiae* YNG318 together with an about 0.9 kb wild-type *Humicola lanuginosa* lipase DNA fragment (see figure 1) prepared from pJSO26 by PCR amplification.

Further, the opened plasmid was also transformed into the yeast recombination host cell alone (*i.e*. without the 0.9 kb synthetic lipase DNA fragment).

The transformed yeast cells were grown as described in the "Materials and Method"-section above, and the transformation frequency was determined as described above.

It was found that the transformation frequency of the opened plasmid alone was very low (10 transformants per mg opened plasmid), in comparison to the transformation frequency of said plasmid/fragment (50,000 transformants per mg opened plasmid).

The plasmid/fragment was PCR amplified resulting in 20 transformants containing fragments covering the lipase gene region of the recombined plasmid/fragments. The recombination mixture of the 20 transformants were analyzed by restriction site digestion using standard methods. The result is displayed in Table 1.

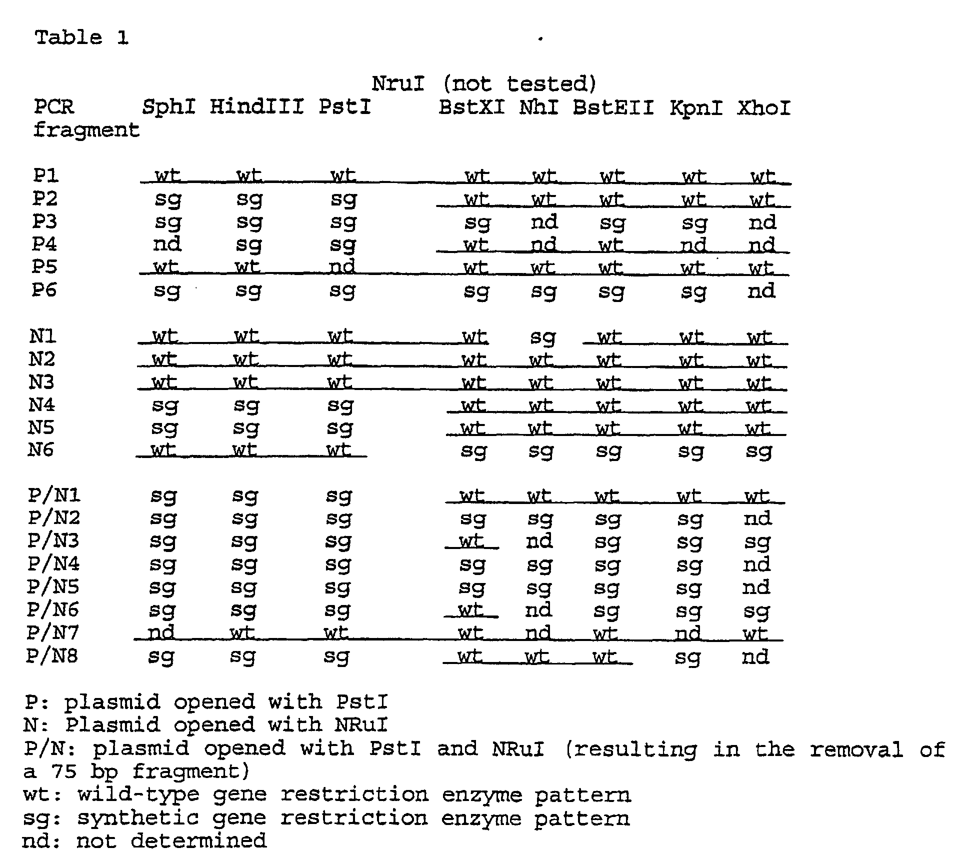

As can bee seen from Table 1 10 transformants (equivalent to 50%) contained recombined DNA sequences. 4 of these 10 DNA sequences (equivalent to 20%) contained either a region of the wild-type gene recombined into the synthetic gene or a region of the synthetic gene recombined into the wild-type fragment.

### Example 2

### In vivo recombination of Humicola lanuginosa lipase variants

The DNA sequences of 20 variants of the *Humicola lanuginosa* lipase were in vivo recombined in the same mixture.

Six vectors were prepared from the lipase variants (a) to (f) (see the list above) by ligation into the yeast expression vector pJSO37. All vectors were cut open with Nrul.

DNA fragment of all 20 homologous DNA sequences (g) to (aa) (see the list above) were prepared by PCR amplification using standard methods.

The 20 DNA fragments and the 6 opened vectors were mixed and transformed into the yeast *Saccharomyces cerevisiae* YNG318 by standard methods. The recombination host cell was cultivated as described above and screened as described above. About 20 transformants were isolated and tested for improved wash performance using the filter assay method described in the "Material and Methods"-section.

Two positive transformants (named A and B) were identified using the filter assay.

In comparison to the wild-type amino acid sequence the two recombined positive transformants had the following mutations.
A is a recombination of two variants.
---- originates from the vector (d)
==== originates from the DNA fragment prepared from variant (y)

B is a recombination of vector (c), DNA fragments (n) and (u).
---- originates from the vector (c)
<<<< originates from the DNA fragment prepared from variant (u)
==== originates from the DNA fragment prepared from variant (n)
???? Amino acid mutation which is not a result of recombination.

As can be seen the resulting positive variants have been formed by recombination two or more variants. The amino acid mutations marked "?????" are not a result of *in vivo* recombination, as none of the shuffled lipase variants (see the list above) comprise any of said mutations. Consequently, these mutations are a result of random mutagenesis arisen during preparation of the DNA fragments by standard PCR amplification.

### Example 3

### Recombination with one frameshift mutantions

Synthetic *Humicola lanuginosa* lipase gene (in vector JSO37) was made inactive at various positions by deleting (positions 184/385) or filling-in (position 290/317/518/746) restriction enzyme sites or by site-directed introduction of a stop codon. All inactive synthetic lipase genes of 900 bp can be deduced from Figure 7).

A number of different 900 bp DNA fragments were made from the above vectors using primer 4699 and primer 5164 using standard PCR technique. Smaller PCR fragments were made using primer 8487 and primer 4548 (260bp), primer 2843 and primer 4548 (488bp).

0.5 ml (app. 0.1 mg) of vectors Blue 425, Blue 426, Blue 428 and Blue 429, opened with Pst I (*i.e.* position 385), vectors Blue 424 and Blue 425 opened with NruI (*i.e.* position 464) were together with 3 ml (app. 0.5 mg) of fragments 424, 425, 426, 428, 429 in varios combination transformed into 100 ml *Sacchromyces cerevisiae* YNG318 competent cells as displayed in Table 1A.

The number of colonies and positives (i.e. with lipase activity) were calculated as the average of 3 plates as described in the Material and Methods section.

The result of the test is shown in Table 1A

**Table 1A**

| *vector + Fragment* | *Number of colonies* | *% of colonies with active lipase activity* |
|---|---|---|
| *1. Blue 428 + 429*◊ | 774 | 16% |
| *2. Blue 429 + 428#* | 645 | 3% |
| *3. Blue 426* + *425#* | 276 | 25% |
| *4. Blue 425 + 426* | 528 | 18% |
| *5. Blue 425*/*Nru I* + *426* | 539 | 28% |
| *6. Blue 425 + 424* | 139 | 7% |
| *7. Blue 424* /*NruI* + *425◊* | 74 | 32% |
| *8. Blue 428 + 425* | 81 | 12% |
| *9. Blue 428 + wt fragment* | 317 | 37% |
| Pairwise recombinations of one frameshift mutation on the vector and another on the fragment on the opposite side of the opening site. ◊ determined by 9 plates; # determined by 6 plates. | | |

The first 2 rows of Table 1A displays vectors and fragments with a frameshift on each side of the PstI site. The "mirror image" experiment in row 2 compared to row 1 gives a reproducible lower number of active colonies. The same is true for row 3 and 4 even though it is not as pronounced. Moving the opening site closer to the frameshift in the vector increases the number of actives as seen in row 5. This can explain the reason for the difference in the "mirror image" experiments. In both cases the higher number of positives has the opening site closer to the frameshift in the vector.

It can therefore be concluded that the closer the mutation is to the end of the vector the higher chance of mixing. This is probably arising from the well known fact that free DNA ends have a high recombinogenic potential. Therefore it is desirable to have as many free DNA ends as possible to increase the mixing of the genes. This is for example obtained in the later example with recombination of multiple overlapping fragments.

Row 6 has a rather low number of actives probably due to the location of the frameshift on the fragment exactly at the PstI opening site of the vector.
Row 7 has the frameshift of the vector close to the opening site and again it gives a high number of actives.

### Recombination with one stop codon mutantions

In order to test if there are any difference in the recombination efficiency of stop codon mutations compared to frameshift mutations the following experiments were made..

The same way as described above 0.5 ml (app. 0.1 mg) vectors Blue 624, Blue 625 and Blue 626 (see Table 1B) opened with PstI comprising stop codons at specified positions (positions 184, 317 and 746, respectively) (perpared by site-directed mutagenesis) were together with 3 ml (app. 0.5 mg) of fragments 624, 625 and 626 transformed into 100 ml *Sacchromyces cerevislae* YNG318 competent cells in varios combination as displayed in Table 1B.

**Table 1B**

| *Vector + Fragment* | *Number of colonies* | *% of colonies with lipase activity* |
|---|---|---|
| *1. Blue 626 + 624* | ND | 40% |
| *2. Blue 624 + 626* | ND | 12% |
| 3. Blue *625 + 624* | ND | 75% |
| 4. Blue 624 + 625 | ND | 10% |
| Pairwise recombinations of one stop codon mutation on the vector and another on the fragment on the opposite side of the opening site. ND = not determined but a high number. | | |

Row 1 and 2 (in Table 1B) have the mutations located at the same place as row 1 and 2 in Table 1A. As can be seen the number of colonies with lipase activity is clearly higher for the stop codon mutations compared to the frameshift mutations, but the same relative difference between the "mirror image" experiments.

This might indicate that the stop codon mutations, which is closer to the "application" of the method, gives a better mixing than frameshift mutations. Row 3 and 4 confirms that the closer the mutation is to the end of the vector the higher chance of mixing.

### Recomhination with one or two frameshift mutation in the vector and one or two frameshift mutations in the fragment

Using the same approach as described above the influence of one or two frameshift mutations in the vector and one or two frameshift mutations in the fragment were tested using vectors Blue 425, 426 and 428 (one mutation) and vectors Blue 442, Blue 443 (two mutations) and fragments 442 and 443 (two frameshift mutations) and fragments 424, 425, 426, 427, 428 (one mutation) and wild-type (no mutation)

The vectors Blue 442 and 443 are double frameshift mutations: Blue 442 = 428+429 and blue 443 = 427+429 (see Figure 7).

Recombination was performed by transforming 0.5 ml vector (app. 0.1 mg) opened with PstI and 3 ml PCR-fragment (app. 0.5 mg) into 100 ml *Sacchromyces cerevisiae* YNG318 competent cells.

The result of the test is shown in Table 2A and Table 2B

**Table 2A**

| Vector + Fragment | Number of colonies | % of colonies with active Lipolase |
|---|---|---|
| 1. Blue 425 + 442 | 142 | 15% |
| 2. Blue 425 + 443 | 144 | 14% |
| 3. Blue 426 + 442 | 42 | 42% |
| 4. Blue 426 + 443# | 77 | 20% |
| 5: Blue 428 + 443 | 115 | 3.8% |
| One frameshift mutation on the vector and two on the fragment on each side of the opening site. # determined by 6 plates. | | |

**Table 2B**

| Vector + Fragment | Number of colonies | % of colonies with active Lipolase |
|---|---|---|
| Blue 442 + 424 | 137 | 0.5% |
| Blue 442 + 426 | 118 | 1.1% |
| Blue 442 + 427# | 125 | 1.3% |
| Blue 443 + 425 | 540 | 2.5% |
| Blue 443 + 426 | 196 | 1.5% |
| Blue 443 + 428 | 469 | 3.1% |
| Blue 442 + wt fragment | 135 | 7.7% |
| Blue 443 + wt fragment | 488 | 10.7% |
| Two frameshift mutations on the vector on each side of the opening site and one on the fragment. # determined by 6 plates. | | |

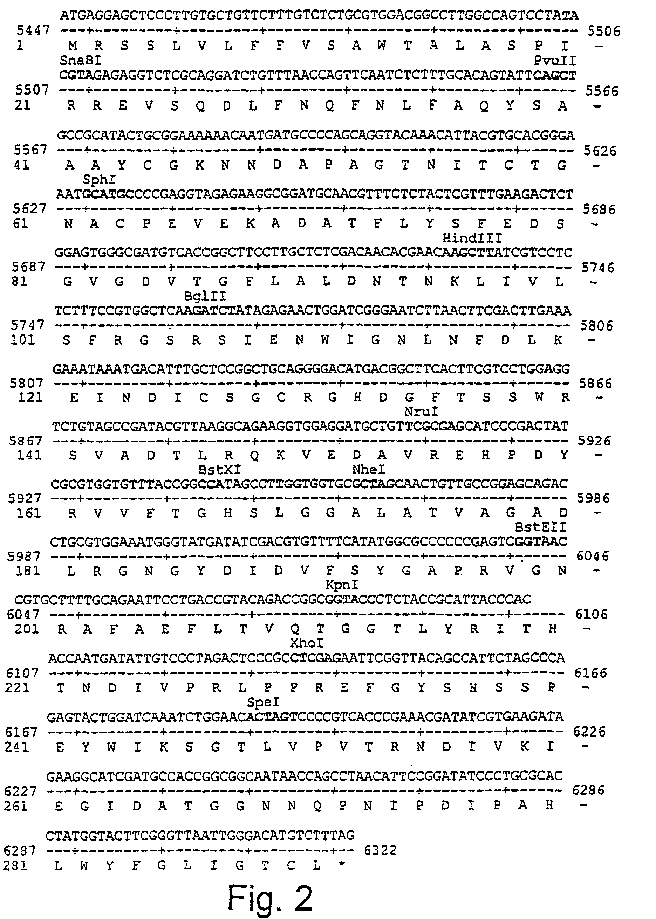

Table 2A shows a rather high number of colonies with lipase activity even with a total of 3 frameshifts (but only one frameshift on the vector) except for the last row where the frameshift on the vector is located far from the opening site. Lane 4 has fewer actives than lane 3 probably due to that the frameshift on the vector is located further away from the opening site than the frameshift on the fragment making the active genes mosaics that are not related to the opening site (see figure 2A). In Table 2B a very low number of actives are observed when there are 2 frameshifts located on the vector. Most of these active colonies are mosaics of the "parent" DNA meaning that the mixing is not related to the opening site (see figure 2B).

### Recombination with two different vectors or fragments

The result of recombination with two different vectors or fragnments the test is shown in Table 3

**Table 3**

| Vector + Fragment | Number of colonies | % of colonies with active Lipolase |
|---|---|---|
| Blue 428/pstI + Blue 429/pst # | 13 | 15% |
| Blue 428/pst + Blue 429/PstI + 442 | 273 | 4.2% |
| Blue 442/pstI + 428 + 429 | 228 | 0.8% |
| Blue 443/pstI + 427 + 428 | 229 | 1.6% |
| Recombinations with 2 different vectors or fragments. # Determined by 1 plate. | | |

A low number of colonies are seen for the control experiment in row 1 of table 3 as expected. The fragment added in the middle row has two frameshifts each corresponding to the frameshift on each vector. Via a tripartite recombination 4.2% actives are created.
With two fragments with each one frameshifts and a vector with the same two frameshifts very few actives are found.

### Recombination with vectors opened at different sites

Opening the vector in one side instead of approximately in the middle still gives good recombination as shown in Table 4. Two vectors opened at different sites can also recombine to some extent (compare with the vector controls in table 13).

**Table 4**

| Vector + Fragment | Number of colonies | % of colonies with active Lipolase |
|---|---|---|
| Blue 428/xho + 429 | 160 | 11% |
| Blue 428/xho+Blue 429/pst# | 35 | 6.3% |
| Opening of the vector in one side instead of in the middle. # determined by 6 plates. | | |

### Recombination at different concentrations of vector and fragment

The relative concentration of vector to fragment do influence the percentage of positive colonies as can be seen in Table 5.

**Table 5**

| Vector + Fragment | Number of colonies | % of colonies with lipase activity |
|---|---|---|
| 0.5µl Blue 426 + 3µl 442 | 42 | 42% |
| 1.5µl Blue 426 + 3µl 442 | 21 | 51% |
| 1.5µl Blue 426 + 9µl 442 | 34 | 26% |
| 1.5µl Blue 426 + 3µl 427 | 230 | 2.8% |
| 1µl Blue 442 + 1µl 425 | 224 | 1.16% |
| 1µl Blue 442 + 2µl 425 | 429 | 0.9% |
| 1µl Blue 442 + 4µl 425 | 434 | 1.6% |
| 1µl Blue 442 + 8µl | 481 | 1.6% |
| 425 | | |
| 1µl Blue 442 + 16µl 425 | 497 | 2.0% |
| Varying the concentration of the vector or fragment. | | |

### Recombination with fragments of different size

The size of the fragment also influences the recombination result as seen in Table 6.

**Table 6**

| Vector + Fragment | Number of colonies | % of colonies with active Lipolase |
|---|---|---|
| Blue 424 + 425 (260bp) | 73 | 34% |
| Blue 424 + 425 (489bp) | 130 | 45% |
| Blue 424 + 424 (480bp) | 133 | 0.3% |
| Blue 424 + 428 (480bp) | 130 | 36% |
| Blue 428 + 425 (480bp) | 150 | 28% |
| Blue 425 + 424 (480bp) | 69 | 0% |
| Blue 425 + 428 (480bp) | 63 | 55% |
| Recombination with smaller fragments than 900 bp. | | |

### Recombination with unopened vectors

Transformation with unopened vectors shows a very low degree of recombination (Table 7).

**Table 7**

| Plasmid | Number of colonies | % of colonies with active Lipolase |
|---|---|---|
| Blue 428 + Blue 429 | 887 | 0.3% |
| Blue 426 + Blue 425 | 697 | 0.7% |
| Recombination of unopened plasmids. | | |

### Example 4

### Test of S. cerevisiae mutants altered in recombination

Using the same approach as described in Example 3 recombination of opened and unopened vectors and fragments were tested using a *Saccharomyces cerevisiae* rad52 mutant as the recombination host cell. The result is displayed in Table 8.

**Table 8**

| Vector + Fragment | Number of colonies | % of colonies with active Lipolase |
|---|---|---|
| Blue 428 + 429 | 0 | 0 |
| Blue 442 + 427 | 0 | 0 |
| Blue 424 + 425 | 0 | 0 |
| Blue 426 + 443 | 0 | 0 |
| Plasmid pJSO 37 | 544 | 100% |
| Recombination result in *rad52* mutant. | | |

The result with *rad52* showed that recombination was completely abolished. The RAD52 function is required for classical recombination (but not for unequal sister-strand mitotic recombination) showing that the recombination of opened vector and fragment could involve a classical recombination mechanism.

### Example 6

### Recombination of multiple partial ping fragments

In order to increase the mixing of the mutations by the recombination method of the invention, recombination of two fragments and one gapped vector were attempted.

**Table 15**

| Vector + Fragment | Number of colonies | % of colonies with lipase activity |
|---|---|---|
| 1. pJSO37/HindIII-XhoI + PCR319+PCR327 | > 2000 | 100% |
| 2. pJSO37/HindIII-XhoI + PCR321+PCR331 | ≈ 2000 | ≈ 0.2% |
| 3. pJSO37/HindIII-XhoI + PCR319+PCR331 | ≈ 1500 | ≈ 1% |
| 4. pJSO37/HindIII-XhoI + PCR319+PCR386 | > 5000 | > 90% |
| 5. pJSO37/HindIII-XhoI + PCR321+PCR386 | > 5000 | ≈ 25% |
| 6. Blue 428/HindIII-XhoI + PCR321+PCR331 | 400 | 0.2% |
| 7. Blue 428/HindIII-XhoI + PCR319+PCR327 | ≈ 1500 | > 90% |
| 8. Blue 428/HindIII-XhoI + PCR321+PCR327 | ≈ 150 | ≈ 10% |
| 9. Blue 428/HindIII-XhoI + PCR327+PCR385 | ≈ 1500 | ≈ 10% |
| 10. Blue 429/HindIII-XhoI + PCR319+PCR386 | ≈ 400 | ≈ 15% |
| 11. Blue 429/HindIII-XhoI + PCR321+PCR386 | ≈ 350 | ≈ 15% |
| 12. Blue 442/HindIII-XhoI + PCR319+PCR327 | ≈ 1500 | ≈ 10% |
| 13. Blue 428/HindIII-XhoI + | 2 | 0 |
| 14. Blue 429/HindIII-XhoI + | 0 | 0 |
| 15. Blue 442/HindIII-XhoI + | 6 | 0 |
| 16. Blue 428/HindIII-XhoI + PCR331 | 4 | 0 |
| 17. Blue 428/HindIII-XhoI + PCR321 | 2 | 0 |
| Recombination result of two fragments and a gapped vector. The last 5 rows are controls. | | |

As can be seen in Table 15, the recovery of the *Humicola lanuginos* lipase gene is very efficient. The last 5 rows in Table 15 shows that the opened vector alone or with only one fragment not covering the whole gap (see figure 3) gives only very few colonies.

The first row is with wild type fragments gives 100% of active colonies.

The second row is with two fragments each containing a frameshift. The fragment PCR331 fragment has the frameshift located at the BglII site which, in this recombination, is not covered by a wild type fragment (see figure 3) and therefore gives about 0% of active lipase. The same is the case for row 3 and 6.

In the row 4, fragment PCR386 containing a frameshift at the SphI site which is overlapped by wild type sequences in the gapped vector. The frameshift was recombined into less than 10% of the genes which is lower than the result for one fragment recombination in the last row of Table 1A above.

In row 5 a rather high mixing is observed between the 2 fragments each containing a frameshift and the wild type gapped vector giving 25% active and 75% inactive lipase colonies. This is probably due to that the fragment PCR321 has the frameshift in the overlap between the 2 fragments and in the gapped region of the vector. If fragment PCR386 contributes to 10% inactives like in row 4, fragment PCR321 gives the remaining 65% inactives - therefore PCR386 gives 35% wt in the overlap.

Row 7 is the "mirror image" of row 4 with the frameshift at the SphI site on the vector (see Figure 7) and 2 wild type fragments giving an integration of the wild type fragment into more than 90% of the vectors.

Row 8 shows like in row 5 that the frameshift of PCR321 in the overlap and gap region gives a very high number of inactive.

In row 9, fragment PCR385 with a frameshift in the vector overlap, causes a very high number of inactives.

Row 10 gives a rather high number of inactives compared to row 7 and 4. It is not increased in row 11.

Row 12 shows that two frameshifts on the vector gives a lower number of actives compared to one in row 7.

The recombination of 3 partial overlapping fragments into a gapped vector is also very efficient as seen in Table 16. The last row with the vector alone gives very few colonies. As can be seen in figure 4 all fragments used are wt. In the first row in table 16, there are rather long overlaps between the vector and fragments, but in the middle row the overlap between PCR353 and 355 is only 10 bp long and it is still very efficiently recombined! This surprising result may be utilized for very easy domain shuffling of even distantly related genes. For example can 3 different domains from 10 different genes be made as PCR fragments, designed to have a 10 to 20 bp overlap by primer design and recombined together and subsequently screened for the best combination (1000 possible combinations).

**Table 16**

| Vector + Fragment | Number of colonies | % of colonies with active Lipolase |
|---|---|---|
| pJSO37/PvuII-SpeI + PCR353+PCR354+PCR367 | > 5000 | 100% |
| pJSO37/PvuII-SpeI + PCR353+PCR355+PCR367 | > 5000 | 100% |
| pJSO37/PvuII-SpeI | 20 | 100% |
| Recombination result of 3 fragments and a gapped vector. The last row is a control. | | |

## Claims

1. A method for preparing polypeptide variants by shuffling different nucleotide sequences of homologous DNA sequences by *in vivo* recombination comprising the steps of
a) forming at least one circular plasmid comprising a DNA sequence encoding a polypeptide,
b) opening said circular plasmid(s) within the DNA sequence(s) encoding the polypeptide(s),
c) preparing at least one DNA fragment comprising a DNA sequence homologous to at least a part of the polypeptide coding region on at least one of the circular plasmid(s), d) introducing at least one of said opened plasmid(s), together with at least one of said homologous DNA fragment(s) covering full-length DNA sequences encoding said polypeptide(s) or parts thereof, into a recombination host cell,
e) cultivating said recombination host cell, and
f) screening for positive polypeptide variants, **characterized in that** two or more opened plasmids are shuffled with one or more homologous DNA fragments in the same shuffling cycle.

2. The method according to claim 1, wherein more than one cycle of step a) to f) are performed.

3. The method according to any of claims 1 or 2, wherein the opened plasmid(s) is(are) gapped.

4. The method according to any of claims 1 to 3 wherein the ratio between the opened plasmid(s) and homologous DNA fragment(s) are in the range from 20:1 to 1:50, preferable from 2:1 to 1:10 (mol vector:mol fragments) with the specific concentrations being from 1 pM to 10 M of the DNA.

5. The method according to any claims 1 to 4, wherein 2 or more, preferably from 2 to 6, especially 2 to 4 of the DNA fragments have partially overlapping regions.

6. The method according to claim 5, wherein the overlapping regions of the DNA fragments lies in the range from 5 to 5000 bp, preferably from 10 bp to 500 bp, especially 10 bp to 100 bp.

7. The method according to any of claims 1 to 6, wherein at least one cycle of step a) to f) is backcrossing with the initially used DNA fragments.

8. The method according to any of claims 1 to 7, wherein the plasmid(s) is (are) opened in the region around the middle of the DNA sequence(s) encoding the polypeptide(s).

9. The method according to any of claims 1 to 8, wherein the plasmid(s) is(are) opened close to a mutation in the DNA sequence(s) encoding the polypeptide(s).

10. The method according to any of claims 1 to 9, wherein the DNA fragment(s) prepared in step c) is(are) prepared under conditions suitable for high, medium or low mutagenesis.

11. The method according to any of claims 1 to 10, wherein the polypeptides producible from the input DNA sequences are enzymes or proteins with biological activity.

12. The method according to claim 11, wherein the polypeptides are enzymes selected from the group including proteases, lipases, cutinases, cellulases, amylases, peroxidases, oxidases and phytases.

13. The method according to claim 11, wherein the polypeptides are proteins with biological activity selected from the group including insulin, ACTH, glucagon, somatostatin, somatotropin, thymosin, parathyroid hormone, pigmentary hormones, somatomedin, erythropoietin, luteinizing hormone, chorionic gonadotropin, hypothalamic releasing factors, antidiuretic hormones, thyroid stimulating hormone, relaxin, interferon, thrombopoietin (TPO) and prolactin.

14. The method according to any of claims 1 to 12, wherein at least one of the initially used input DNA sequences is a wild-type DNA sequence, such as a DNA sequence coding for wild-type enzymes, in particular lipases, derived from filamentous fungi, such as *Humicola* sp., in particular *Humicola lanuginosa,* especially *Humicola lanuginosa* DSM 4109.

15. The method according to claim 14, wherein at least one of the input DNA sequences is selected from the group of vectors (a) to (f) and/or DNA fragments (g) to (aa) coding for *Humicola lanuginosa*, lipase variants.

16. The method according to any of claims 1 to 12, wherein at least one of the initially used input DNA sequences is a wild-type DNA sequence, such as a DNA sequence coding for wild-type enzymes, in particular lipases, derived from filamentous fungi of the genera *Absidia, Rhizopus, Emericella, Aspergillus, Penicillium, Eupenicillium, Paecilomyces, Talaromyces, Thermoascus* and *Sclerocleista.*

17. The method according to any of claims 1 to 12, wherein at least one of the initially used input DNA sequences is a wild-type DNA sequence, such as a DNA sequence coding for wild-type enzymes, in particular lipases, derived from bacteria, such as *Pseudomonas* sp., in particular *Ps. fragi, Ps. stutzeri, Ps. cepacia, Ps. fluorescens, Ps. plantarii, Ps. gladioli, Ps. alcaligenes, Ps. pseudoalcaligenes, Ps. mendocina, Ps. auroginosa, Ps. glumae, Ps. syringae, Ps. wisconsinensis, or a* strain of *Bacillus* sp., in particular B. *subtilis, B. stearothermophilus* or or *B. pumilus, or* or a strain of *Streptomyces* sp., in particular *S. scabies,* or a strain of *Chromobacterium* sp. *in particular C. viscosum.*

18. The method according to any of claims 1 to 12, wherein at least one of the initially used input DNA sequences is a variant DNA sequence, such as a DNA sequence coding for a variant enzyme, in particular lipase variants, derived from yeasts, such as *Candida* sp., in particular *Candida rugosa,* or Geotrichum *sp.*, in particular *Geotrichum candidum.*

19. The method according to any of claims 1 to 18, wherein the homologous input DNA sequences are at least 60%, preferably at least 70%, better more than 80%, especially more than 90%, and even up to 100% homologous.

20. The method according to any of claims 1 to 19, wherein the recombination host cell is a eukaryotic cell, such as a fungal cell or a plant cell.

21. The method according to claim 20, wherein said fungal cell is a yeast cell from the group of cell of *Saccharomyces* sp., in particular strains of *Saccharomyces cerevisiae* or *Saccharomyces kluyveri* or *Schizosaccharomyces* sp., in particular *Schizosaccharomyces pombe, or Kluyveromyces* sp., such as *K. lactis,* or *Hansenula sp.,* in particular *H. polymorpha, or Pichia* sp., in particular *P. pastoris,* or a filamentous fungi from the group of *Aspergillus* sp., in particular *A. niger, A. nidulans* or *A. oryzae,* or *Neurospora* sp., or *Fusarium* sp., in particular *F. oxysporum, or Trichodelma sp..*

22. The method according to any of claims 1 to 21, wherein the plasmid DNA sequence(s) coding for the polypeptide(s) is(are) operably linked to a replication sequence.

23. The method according to claim 22, wherein the plasmid DNA sequence(s) encoding the polypeptide(s) is(are) operably linked to a functional promoter sequence.

24. The method according to claim 23, wherein the plasmid is an expression plasmid.

25. The method according to claim 24, wherein the expression plasmid is pJSO26 or pJSO37.

## Patentansprüche

1. Verfahren zur Herstellung von Polypeptidvarianten durch Mischen von verschiedenen Nukleotidsequenzen homologer DNA-Sequenzen durch *in vivo* Rekombination, umfassend die Schritte
a) Bilden mindestens eines ringförmigen Plasmids umfassend eine DNA-Sequenz kodierend für ein Polypeptid,
b) Öffnen des ringförmigen Plasmids/der ringförmigen Plasmide innerhalb der DNA-Sequenz/en, kodierend für das Polypeptid/die Polypeptide,
c) Herstellen mindestens eines DNA-Fragments umfassend eine DNA-Sequenz, die mindestens zu einem Teil der Polypeptid-kodierenden Regionen auf mindestens einem des ringförmigen Plasmids/der ringförmigen Plasmide homolog ist,
d) Einführen mindestens eines geöffneten Plasmids/der geöffneten Plasmide, zusammen mit mindestens einem der homologen DNA-Fragmente/der homologen DNA-Fragmente, dass bzw. die Gesamtlängen-DNA-Sequenzen abdeckt, welches das Polypeptid/die Polypeptide oder Teile davon koliert, in eine Rekombinationswirtszelle,
e) Kultivieren der Rekombinationswirtszelle, und
f) Screenen nach positiven Polypeptidvarianten, **dadurch gekennzeichnet, dass** 2 oder mehr geöffnete Plasmide mit einem oder mehreren homologen DNA-Fragmenten in demselben Mischzyklus vermischt werden.

2. Verfahren nach Anspruch 1, wobei mehr als ein Zyklus von Schritt a) bis f) durchgeführt wird.

3. Verfahren nach einem der Ansprüche 1 oder 2, wobei das geöffnete Plasmid/die geöffneten Plasmide gespalten ist/sind.

4. Verfahren nach einem der Ansprüche 1-3, wobei das Verhältnis zwischen dem geöffneten Plasmid/den geöffneten Plasmiden und dem homologen DNA-Fragment/den homologen DNA-Fragmenten im Bereich von 20:1 bis 1:50, beträgt vorzugsweise von 2:1 bis 1:10 (molvektor:molfragmente) mit spezifischen Konzentrationen von 1 pM bis 10 M der DNA.

5. Verfahren nach einem der Ansprüche 1-4, wobei 2 oder mehr, vorzugsweise 2-6, insbesondere 2-4 der DNA-Fragmente teilweise überlappende Regionenaufweisen.

6. Verfahren nach Anspruch 5, wobei die überlappenden Regionen der DNA-Fragmente im Bereich von 5 bis 5000 bp, vorzugsweise von 10 bp bis 500 bp, insbesondere 10 bp bis 100 bp liegen.

7. Verfahren nach einem der Ansprüche 1-6, wobei mindestens ein Zyklus der Schritt a) bis f) mit den Anfangs benutzten DNA-Fragmenten zurückgekreuzt wird.

8. Verfahren nach einem der Ansprüche 1-7, wobei das Plasmid/die Plasmide in der Region rund um die Mitte der DNA-Sequenz/en kodierend das Polypeptid/die Polypeptide geöffnet wird/werden.

9. Verfahren nach einem der Ansprüche 1-8, wobei das Plasmid/die Plasmide in der Nähe einer Mutation in der DNA-Sequenz/en kodierend das Poylpeptide/die Polypeptide geöffnet wird/werden.

10. Verfahren nach einem der Ansprüche 1-9, wobei das DNA-Fragment/die DNA-Fragmente, hergestellt in Schritt c) unter Bedingungen, die geeignet sind für hohe, mittlere oder niedrige Mutagenese hergestellt wird/werden.

11. Verfahren nach einem der Ansprüche 1-10, wobei die Polypeptide herstellbar aus den Ausgangs-DNA-Sequenzen Enzyme oder Proteine mit biologischer Aktivität sind.

12. Verfahren nach Anspruch 11, wobei die Polypeptide Enzyme sind, die ausgewählt sind aus der Gruppe umfassend Protease, Lipasen, Cutinasen, Cellulasen, Amylasen, Peroxidasen, Oxidasen und Phytasen.

13. Verfahren nach Anspruch 11, wobei die Polypeptide Proteine mit biologischer Aktivität sind, die ausgewählt sind aus der Gruppe umfassend Insulin, ACTH, Glucagon, Samatostatin, Samatotropin, Thymosin, Parathyroidhormon, Pigmenthormone, Samatomedin, Erythropoietin, Luteinisierungshormon, Choriongonadotropin, Hypothalamische Freisetzungsfaktoren, harnhemmende Hormone, Thyroidstimulierende Hormone, Relaxin, Interferon, Thrombopoietin (TPO) und Prolactin.

14. Verfahren nach einem der Ansprüche 1-12, wobei mindestens eine der anfangs benutzten Ausgangs-DNA-Sequenzen eine Wildtyp DNA-Sequenz ist, eine solche DNA-Sequenz für Wildtyp-Enzyme kodiert, insbesondere Lipasen, abgeleitet von filamentösen Pilzen wie *Humicola* sp., insbesondere *Humicola lanuginosa,* vor allem *Humicola lanuginosa* DSM 4109.

15. Verfahren nach Anspruch 14, wobei mindestens eine der Ausgangs-DNA-Sequenzen ausgewählt ist aus der Gruppe von Vektoren (a) bis (f) und/oder DNA-Fragmenten (g) bis (aa) kodierend *Humicola lanuginosa* Lipasevarianten.

16. Verfahren nach einem der Ansprüche 1-12, wobei mindestens eine der anfangs benutzten Ausgangs-DNA-Sequenzen eine Wildtyp-DNA-Sequenz ist, wie eine DNA-Sequenz kodierend für Wildtyp-Enzyme, insbesondere Lipasen, abgeleitet von filamentösen Pilzen der Gattung *Absidia, Rhizopus, Emericella, Aspergillus, Penicillium, Eupenicillium, Paecilomyces, Talaromyces, Thermoascus* und *Sclerocleista.*

17. Verfahren nach einem der Ansprüche 1-12, wobei mindestens eine der anfangs benutzten Ausgangs-DNA-Sequenzen eine Wildtyp-DNA-Sequenz ist, wie eine DNA-Sequenz kodierend für Wildtypenzyme, insbesondere Lipasen, abgeleitet von Bakterien wie *Pseudomonas* sp., insbesondere *Ps. fragi, Ps. stutzeri, Ps. cepacia, Ps. fluorescens, Ps. plantarii*, *Ps. gladioli, Ps. alcaligenes, Ps. pseudoalcaligenes, Ps. mendocina, Ps. auroginosa, Ps. glumae, Ps. syringae, Ps. wisconsinensis*, oder ein Stamm von *Bacillus* sp., insbesondere *B. subtilis, B. stearothermophilus,* oder *B. pumilus,* oder ein Stamm von *Streptomyces* sp., insbesondere *S. scabies,* oder ein Stamm von *Chromobacterium* sp. insbesondere *C. viscosum.*

18. Verfahren nach einem der Ansprüche 1-12, wobei mindestens eine der anfangs benutzten Ausführungs-DNA-Sequenzen eine DNA-Sequenzvariante ist, wie eine DNA-Sequenz kodierend für eine Enzymvariante, insbesondere Lipasevarianten, abgeleitet von Hefen, wie *Candida* sp., insbesondere *Candida rugosa,* oder *Geotrichum* sp., insbesondere *Geotrichum candidum.*

19. Verfahren nach einem der Ansprüche 1-18, wobei die homologen Ausgangs-DNA-Sequenzen mindestens 60%, vorzugsweise mindestens 70% weiter bevorzugt mindestens 80%, vor allem mehr als 90% und sogar bis zu 100% homolog sind.

20. Verfahren nach einem der Ansprüche 1-19, wobei die Rekombinations-Wirtszelle eine eukaryonte Zelle ist, wie eine Pilzzelle oder eine Pflanzenzelle.

21. Verfahren nach Anspruch 20, wobei die Pilzzelle eine Hefezelle aus der Gruppe von Zellen von Saccharomyces sp., insbesondere Stämmen von *Saccharomyces cerevisiae* oder *Saccharomyces kluyveri* oder *Schizosaccharomyces* sp., insbesondere *Schizosaccharomyces pombe,* oder *Kluyveromyces* sp., wie *K*. *lactis,* oder *Hansenula* sp., insbesondere *H. polymorpha,* oder *Pichia* sp., insbesondere *P. pastoris*, oder ein filamentöser Pilz aus der Gruppe von *Aspergillus* sp., insbesondere *A. niger, A. nidulans* oder *A. oryzae,* oder *Neurospora* sp., oder *Fusarium* sp., insbesondere *F. oxysporum*, oder *Trichoderma* sp. ist.

22. Verfahren nach einem der Ansprüche 1-21, wobei die Plasmid-DNA-Sequenz/en kodierend für das Polypeptid/die Polypeptide operativ mit einer Replikationssequenz verbunden ist/sind.

23. Verfahren nach Anspruch 22, wobei die Plasmid-DNA-Sequenz/en kodierend das Polypeptid/die Polypeptide operativ mit einer funktionalen Promotorsequenz verbunden ist/sind.

24. Verfahren nach Anspruch 23, wobei das Plasmid ein Expressionsplasmid ist.

25. Verfahren nach Anspruch 24, wobei das Expresssionsplasmid pJSO26 oder pJSO37 ist.

## Revendications

1. Procédé pour préparer des variants polypeptidiques en entremêlant différentes séquences nucléotidiques de séquences d'ADN homologues par une recombinaison *in vivo,* comportant les étapes consistant à :
(a) former au moins un plasmide circulaire comportant une séquence d'ADN codant pour un polypeptide,
(b) ouvrir ledit ou lesdits plasmides circulaires dans la ou les séquences d'ADN codant pour le ou les polypeptides,
(c) préparer au moins un fragment d'ADN comportant une séquence d'ADN homologue à au moins une partie de la région codante polypeptidique sur au moins un des plasmides circulaires,
(d) introduire au moins un desdits plasmides ouverts, ensemble avec au moins desdits fragments d'ADN homologues couvrant des séquences d'ADN de longueur totale codant pour le ou les polypeptides ou des parties de celles-ci, dans une cellule hôte de recombinaison,
(e) cultiver ladite cellule hôte de recombinaison, et
(f) cribler des variants polypeptidiques positifs, **caractérisé en ce que** deux ou plus de deux plasmides ouverts sont entremêlés avec un ou plusieurs fragments d'ADN homologues dans le même cycle d'entremêlement.

2. Procédé selon la revendication 1, dans lequel plus d'un cycle constitué des étapes a) à f) sont effectués.

3. Procédé selon l'une quelconque des revendications 1 ou 2, dans lequel le ou les plasmides ouverts sont ouverts en grand.

4. Procédé selon l'une quelconque des revendications 1 à 3, dans lequel le rapport entre le ou les plasmides ouverts et le ou les fragments d'ADN homologues se trouve dans la plage allant de 20:1 à 1:50, de préférence de 2:1 à 1:10 (vecteur en mole: fragments en mole) avec les concentrations spécifiques qui sont de 1 pM à 10 M de l'ADN.

5. Procédé selon l'une quelconque des revendications 1 à 4, dans lequel 2 ou plus de 2, de préférence de 2 à 6, en particulier de 2 à 4, des fragments d'ADN ont des régions partiellement chevauchantes.

6. Procédé selon la revendication 5, dans lequel les régions chevauchantes des fragments d'ADN se trouvent dans la plage allant de 5 à 5000 pb, de préférence de 10 pb à 500 pb, en particulier de 10 pb à 100 pb.

7. Procédé selon l'une quelconque des revendications 1 à 6, dans lequel au moins un cycle constitué des étapes a) à f) effectue un rétrocroisement avec les fragments d'ADN initialement utilisés.

8. Procédé selon l'une quelconque des revendications 1 à 7, dans lequel le ou les plasmides sont ouverts dans la région autour du milieu de la ou les séquences d'ADN codant pour le ou les polypeptides.

9. Procédé selon l'une quelconque des revendications 1 à 8, dans lequel le ou les plasmides sont ouverts à proximité d'une mutation dans la ou les séquences d'ADN codant pour le ou les polypeptides.

10. Procédé selon l'une quelconque des revendications 1 à 9, dans lequel le ou les fragments d'ADN préparés à l'étape c) sont préparés sous des conditions adaptées pour une mutagenèse forte, modérée ou faible.

11. Procédé selon l'une quelconque des revendications 1 à 10, dans lequel les polypeptides pouvant être produits à partir des séquences d'ADN d'entrée sont des enzymes ou des protéines ayant une activité biologique.

12. Procédé selon la revendication 11, dans lequel les polypeptides sont des enzymes sélectionnées parmi le groupe incluant des protéases, lipases, cutinases, cellulases, amylases, peroxydases, oxydases et phytases.

13. Procédé selon la revendication 11, dans lequel les polypeptides sont des protéines ayant une activité biologique sélectionnées parmi le groupe incluant de l'insuline, ACTH, glucagon, somatostatine, somatotropine, thymosine, hormone parathyroïde, hormone pigmentaire, somatomédine, érythropoïétine, hormone lutéinisante, gonadotropine chorionique, facteurs de libération hypothalamique, hormone antidiurétique, hormone de stimulation thyroïde, relaxine, interféron, thrombopoïétine (TPO) et prolactine.

14. Procédé selon l'une quelconque des revendications 1 à 12, dans lequel au moins une des séquences d'ADN d'entrée initialement utilisées est une séquence d'ADN de type sauvage, telle qu'une séquence d'ADN codant pour des enzymes de type sauvage, en particulier des lipases, dérivées de champignons filamenteux, tels que Hu*micola* sp., en particulier *Humicola lanuginosa,* en particulier *Humicola lanuginosa* DSM 4109.

15. Procédé selon la revendication 14, dans lequel au moins une des séquences d'ADN d'entrée est sélectionnée parmi le groupe des vecteurs (a) à (f) et/ou des fragments d'ADN (g) à (aa) codant pour des variants de lipase *d'Humicola lanuginosa.*

16. Procédé selon l'une quelconque des revendications 1 à 12, dans lequel au moins une des séquences d'ADN d'entrée initialement utilisées est une séquence d'ADN de type sauvage, telle qu'une séquence d'ADN codant pour des enzymes de types sauvage, en particulier des lipases, dérivées de champignons filamenteux du genre *Absidia, Rhizopus, Emericella, Aspergillus, Pénicillium, Eupenicillium, Paecilomyces, Talaromyces, Thermoascus* et *Sclerocleista.*

17. Procédé selon l'une quelconque des revendications 1 à 12, dans lequel au moins une des séquences d'ADN d'entrée initialement utilisées est une séquence d'ADN de type sauvage, telle qu'une séquence d'ADN codant pour des enzymes de type sauvage, en particulier des lipases, dérivées de bactéries, telles que *Pseudomonas sp.,* en particulier *Ps. fragi, Ps. stutzeri, Ps. cepacia, Ps. fluorescens, Ps. plantarii, Ps. gladioli, Ps. alcaligenes, Ps. pseudoalcaligenes, Ps. mendocina, Ps. auroginosa, Ps. glumae, Ps. syringae, Ps. wisconsinensis,* ou une souche de *Bacillus* sp., en particulier *B. subtilis, B. stearothermophilus* ou *B. pumilus,* ou une souche de *Streptomyces* sp., en particulier *S. scabies,* ou une souche de *Chromobacterium* sp. en particulier *C. viscosum.*

18. Procédé selon l'une quelconque des revendications 1 à 12, dans lequel au moins une des séquences d'ADN d'entrée initialement utilisées est une séquence d'ADN variante, telle qu'une séquence d'ADN codant pour une enzyme variante, en particulier des variants de lipase, dérivés de levures, telles que *Candida sp.,* en particulier *Candida rugosa,* ou *Geotrichum sp.,* en particulier *Geotrichum candidum.*

19. Procédé selon l'une quelconque des revendications 1 à 18, dans lequel les séquences d'ADN d'entrée homologues sont homologues à au moins 60 %, de préférence au moins 70 %, de manière plus préférée à plus de 80 %, en particulier plus de 90 %, et même jusqu'à 100 %.

20. Procédé selon l'une quelconque des revendications 1 à 19, dans lequel la cellule hôte de recombinaison est une cellule eucaryote, telle qu'une cellule fongique ou une cellule végétale.

21. Procédé selon la revendication 20, dans lequel ladite cellule fongique est une cellule de levure provenant du groupe de cellule de *Saccharomyces* sp., en particulier des souches de *Saccharomyces cerevisiae* ou *Saccharomyces kluyveri* ou *Schizosaccharomyces* sp., en particuier *Schizosaccharomyces pombe*, ou *Kluyveromyces* sp., telles que *K. lactis* ou *Hansenula* sp., en particulier *H. polymorpha*, ou *Pichia* sp., en particulier P. *pastoris*, ou un champignon filamenteux provenant du groupe de *Aspergillus* sp., en particulier *A. niger, A. nidulans* ou *A. oryaze,* ou *Neurospora* sp., *ou Fusarium* sp., en particulier F. *oxysporum*, ou *Trichoderma* sp.

22. Procédé selon l'une quelconque des revendications 1 à 21, dans lequel la ou les séquences d'ADN plasmidiques codant pour le ou les polypeptides sont liées de manière opérationnelle à une séquence de réplication.

23. Procédé selon la revendication 22, dans lequel la ou les séquences d'ADN plasmidiques codant pour le ou les polypeptides sont liées de manière opérationnelle à une séquence promoteur fonctionnelle.

24. Procédé selon la revendication 23, dans lequel le plasmide est un plasmide d'expression.

25. Procédé selon la revendication 24, dans lequel le plasmide d'expression est pJS026 ou pJS037.


### SEQUENCE LISTING

(1) GENERAL INFORMATION:
   (i) APPLICANT:
      (A) NAME: Novo Nordisk A/S
      (B) STREET: Novo Alle
      (C) CITY: Bagsvaerd
      (E) COUNTRY: Denmark
      (F) POSTAL CODE (ZIP): DK-2880
      (G) TELEPHONE: +45 4444 8888
      (H) TELEFAX: +45 4449 3256
   (ii)TITLE OF INVENTION: Method for preparing polypeptide variants
   (iii) NUMBER OF SEQUENCES: 15
   (iv) (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.30B (EPO)
(2) INFORMATION FOR SEQ ID NO: 1:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "Primer 2843"
   (xi) SEQUENCE DESCRIPTION: SEQ ID NO: 1:
   ACAAACATTA CGTGCACGGG 20
(2) INFORMATION FOR SEQ ID NO: 2:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "Primer 4699"
   (xi) SEQUENCE DESCRIPTION: SEQ ID NO: 2:
   CGGTACCCGG GGATCCAC 18
(2) INFORMATION FOR SEQ ID NO: 3:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "Primer 5164"
   (xi) SEQUENCE DESCRIPTION: SEQ ID NO: 3:
   AATTACATCA TGCGGCCC 18
(2) INFORMATION FOR SEQ ID NO: 4:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "Primer 8487"
   (xi) SEQUENCE DESCRIPTION: SEQ ID NO: 4:
   CATTTGCTCC GGCTGCAGGG A 21
(2) INFORMATION FOR SEQ ID NO: 5:
   (i) SEQUENCE CHARACTERISTICS:
      (A) LENGTH: 60 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "Primer 4548"
   (xi) SEQUENCE DESCRIPTION: SEQ ID NO: 5:
(2) INFORMATION FOR SEQ ID NO: 6:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "Primer 5576"
   (xi) SEQUENCE DESCRIPTION: SEQ ID NO: 6:
   GGTCTGTACG GTCAGGAATT C 21
(2) INFORMATION FOR SEQ ID NO: 7:
   (i) SEQUENCE CHARACTERISTICS:
      (A) LENGTH: 19 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "Primer 5578"
   (xi) SEQUENCE DESCRIPTION: SEQ ID NO: 7:
   CGTTTCGGGT GACGGGGAC 19
(2) INFORMATION FOR SEQ ID NO: 8:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "Primer 1596"
   (xi) SEQUENCE DESCRIPTION: SEQ ID NO: 8:
   GGAGCAAATG TCATTTAT 18
(2) INFORMATION FOR SEQ ID NO: 9:
   (i) SEQUENCE CHARACTERISTICS:
      (A) LENGTH: 64 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "Primer 4545"
   (xi) SEQUENCE DESCRIPTION: SEQ ID NO: 9:
(2) INFORMATION FOR SEQ ID NO: 10:
   (i) SEQUENCE CHARACTERISTICS:
      (A) LENGTH: 876 base pairs
      (B) TYPE: nucleic acid
      (C) STRANDEDNESS: single
      (D) TOPOLOGY: circular
   (ii) MOLECULE TYPE: other nucleic acid
      (A) DESCRIPTION: /desc = "Vector pJSO26"
   (vi) ORIGINAL SOURCE:
      (B) STRAIN: Humicola lanuginosa
   (ix) FEATURE:
      (A) NAME/KEY: CDS
      (B) LOCATION:1..876
   (xi) SEQUENCE DESCRIPTION: SEQ ID NO: 10:
   ATG AGG AGC TCC CTT GTG CTG TTC TTT GTC TCT GCG TGG ACG GCC TTG       48
   Met Arg Ser Ser Leu Val Leu Phe Phe Val Ser Ala Trp Thr Ala Leu
    1               5                  10                  15
   GCC AGT CCT ATT CGT CGA GAG GTC TCG CAG GAT CTG TTT AAC CAG TTC       96
   Ala Ser Pro Ile Arg Arg Glu Val Ser Gln Asp Leu Phe Asn Gln Phe
               20                  25                  30
   AAT CTC TTT GCA CAG TAT TCT GCA GCC GCA TAC TGC GGA AAA AAC AAT      144
   Asn Leu Phe Ala Gln Tyr Ser Ala Ala Ala Tyr Cys Gly Lys Asn Asn
           35                  40                  45
   GAT GCC CCA GCT GGT ACA AAC ATT ACG TGC ACG GGA AAT GCC TGC CCC      192
   Asp Ala Pro Ala Gly Thr Asn Ile Thr Cys Thr Gly Asn Ala Cys Pro
       50                  55                  60
   GAG GTA GAG AAG GCG GAT GCA ACG TTT CTC TAC TCG TTT GAA GAC TCT      240
   Glu Val Glu Lys Ala Asp Ala Thr Phe Leu Tyr Ser Phe Glu Asp Ser
   65                  70                  75                  80
   GGA GTG GGC GAT GTC ACC GGC TTC CTT GCT CTC GAC AAC ACG AAC AAA      288
   Gly Val Gly Asp Val Thr Gly Phe Leu Ala Leu Asp Asn Thr Asn Lys
                   85                  90                  95
   TTG ATC GTC CTC TCT TTC CGT GGC TCT CGT TCC ATA GAG AAC TGG ATC      336
   Leu Ile Val Leu Ser Phe Arg Gly Ser Arg Ser Ile Glu Asn Trp Ile
               100                 105                 110
   GGG AAT CTT AAC TTC GAC TTG AAA GAA ATA AAT GAC ATT TGC TCC GGC      384
   Gly Asn Leu Asn Phe Asp Leu Lys Glu Ile Asn Asp Ile Cys Ser Gly
           115                 120                 125
   TGC AGG GGA CAT GAC GGC TTC ACT TCG TCC TGG AGG TCT GTA GCC GAT        432
   Cys Arg Gly His Asp Gly Phe Thr Ser Ser Trp Arg Ser Val Ala Asp
       130                 135                 140
   ACG TTA AGG CAG AAG GTG GAG GAT GCT GTG AGG GAG CAT CCC GAC TAT        480
   Thr Leu Arg Gln Lys Val Glu Asp Ala Val Arg Glu His Pro Asp Tyr
   145                 150                 155                 160
   CGC GTG GTG TTT ACC GGA CAT AGC TTG GGT GGT GCA TTG GCA ACT GTT        528
   Arg Val Val Phe Thr Gly His Ser Leu Gly Gly Ala Leu Ala Thr Val
                   165                 170                 175
   GCC GGA GCA GAC CTG CGT GGA AAT GGG TAT GAT ATC GAC GTG TTT TCA        576
   Ala Gly Ala Asp Leu Arg Gly Asn Gly Tyr Asp Ile Asp Val Phe Ser
               180                 185                 190
   TAT GGC GCC CCC CGA GTC GGA AAC AGG GCT TTT GCA GAA TTC CTG ACC        624
   Tyr Gly Ala Pro Arg Val Gly Asn Arg Ala Phe Ala Glu Phe Leu Thr
           195                 200                 205
   GTA CAG ACC GGC GGA ACA CTC TAC CGC ATT ACC CAC ACC AAT GAT ATT        672
   Val Gln Thr Gly Gly Thr Leu Tyr Arg Ile Thr His Thr Asn Asp Ile
       210                 215                 220
   GTC CCT AGA CTC CCG CCG CGC GAA TTC GGT TAC AGC CAT TCT AGC CCA        720
   Val Pro Arg Leu Pro Pro Arg Glu Phe Gly Tyr Ser His Ser Ser Pro
   225                 230                 235                 240
   GAG TAC TGG ATC AAA TCT GGA ACC CTT GTC CCC GTC ACC CGA AAC GAT        768
   Glu Tyr Trp Ile Lys Ser Gly Thr Leu Val Pro Val Thr Arg Asn Asp
                   245                 250                 255
   ATC GTG AAG ATA GAA GGC ATC GAT GCC ACC GGC GGC AAT AAC CAG CCT        816
   Ile Val Lys Ile Glu Gly Ile Asp Ala Thr Gly Gly Asn Asn Gln Pro
               260                 265                 270
   AAC ATT CCG GAT ATC CCT GCG CAC CTA TGG TAC TTC GGG TTA ATT GGG        864
   Asn Ile Pro Asp Ile Pro Ala His Leu Trp Tyr Phe Gly Leu Ile Gly
           275                 280                 285
   ACA TGT CTT TAG                                                        876
   Thr Cys Leu  *
       290
(2) INFORMATION FOR SEQ ID NO: 11:
   (i) SEQUENCE CHARACTERISTICS:
      (A) LENGTH: 292 amino acids
      (B) TYPE: amino acid
      (D) TOPOLOGY: linear
   (ii) MOLECULE TYPE: protein
   (xi) SEQUENCE DESCRIPTION: SEQ ID NO: 11:
   Met Arg Ser Ser Leu Val Leu Phe Phe Val Ser Ala Trp Thr Ala Leu
     1               5                  10                  15
   Ala Ser Pro Ile Arg Arg Glu Val Ser Gln Asp Leu Phe Asn Gln Phe
                20                  25                  30
   Asn Leu Phe Ala Gln Tyr Ser Ala Ala Ala Tyr Cys Gly Lys Asn Asn
            35                  40                  45
   Asp Ala Pro Ala Gly Thr Asn Ile Thr Cys Thr Gly Asn Ala Cys Pro
        50                  55                  60
   Glu Val Glu Lys Ala Asp Ala Thr Phe Leu Tyr Ser Phe Glu Asp Ser
    65                  70                  75                  80
   Gly Val Gly Asp Val Thr Gly Phe Leu Ala Leu Asp Asn Thr Asn Lys
                    85                  90                  95
   Leu Ile Val Leu Ser Phe Arg Gly Ser Arg Ser Ile Glu Asn Trp Ile
               100                 105                 110
   Gly Asn Leu Asn Phe Asp Leu Lys Glu Ile Asn Asp Ile Cys Ser Gly
           115                 120                 125
   Cys Arg Gly His Asp Gly Phe Thr Ser Ser Trp Arg Ser Val Ala Asp
       130                 135                 140
   Thr Leu Arg Gln Lys Val Glu Asp Ala Val Arg Glu His Pro Asp Tyr
   145                 150                 155                 160
   Arg Val Val Phe Thr Gly His Ser Leu Gly Gly Ala Leu Ala Thr Val
                   165                 170                 175
   Ala Gly Ala Asp Leu Arg Gly Asn Gly Tyr Asp Ile Asp Val Phe Ser
               180                 185                 190
   Tyr Gly Ala Pro Arg Val Gly Asn Arg Ala Phe Ala Glu Phe Leu Thr
           195                 200                 205
   Val Gln Thr Gly Gly Thr Leu Tyr Arg Ile Thr His Thr Asn Asp Ile
       210                 215                 220
   Val Pro Arg Leu Pro Pro Arg Glu Phe Gly Tyr Ser His Ser Ser Pro
   225                 230                 235                 240
   Glu Tyr Trp Ile Lys Ser Gly Thr Leu Val Pro Val Thr Arg Asn Asp
                   245                 250                 255
   Ile Val Lys Ile Glu Gly Ile Asp Ala Thr Gly Gly Asn Asn Gln Pro
               260                 265                 270
   Asn Ile Pro Asp Ile Pro Ala His Leu Trp Tyr Phe Gly Leu Ile Gly
           275                 280                 285
   Thr Cys Leu  *
       290
(2) INFORMATION FOR SEQ ID NO: 12:
   (i) SEQUENCE CHARACTERISTICS:
      (A) LENGTH: 876 base pairs
      (B) TYPE: nucleic acid
      (C) STRANDEDNESS: single
      (D) TOPOLOGY: circular
   (ii) MOLECULE TYPE: other nucleic acid
      (A) DESCRIPTION: /desc = "Vector pJSO37"
   (vi) ORIGINAL SOURCE:
      (B) STRAIN: *Humicola lanuginosa*
   (ix) FEATURE:
      (A) NAME/KEY: CDS
      (B) LOCATION:1..876
   (xi) SEQUENCE DESCRIPTION: SEQ ID NO: 12:
   ATG AGG AGC TCC CTT GTG CTG TTC TTT GTC TCT GCG TGG ACG GCC TTG      48
   Met Arg Ser Ser Leu Val Leu Phe Phe Val Ser Ala Trp Thr Ala Leu
    1               5                  10                  15
   GCC AGT CCT ATA CGT AGA GAG GTC TCG CAG GAT CTG TTT AAC CAG TTC      96
   Ala Ser Pro Ile Arg Arg Glu Val Ser Gln Asp Leu Phe Asn Gln Phe
               20                  25                  30
   AAT CTC TTT GCA CAG TAT TCA GCT GCC GCA TAC TGC GGA AAA AAC AAT     144
   Asn Leu Phe Ala Gln Tyr Ser Ala Ala Ala Tyr Cys Gly Lys Asn Asn
           35                  40                  45
   GAT GCC CCA GCA GGT ACA AAC ATT ACG TGC ACG GGA AAT GCA TGC CCC     192
   Asp Ala Pro Ala Gly Thr Asn Ile Thr Cys Thr Gly Asn Ala Cys Pro
       50                  55                  60
   GAG GTA GAG AAG GCG GAT GCA ACG TTT CTC TAC TCG TTT GAA GAC TCT     240
   Glu Val Glu Lys Ala Asp Ala Thr Phe Leu Tyr Ser Phe Glu Asp Ser
   65                  70                  75                  80
   GGA GTG GGC GAT GTC ACC GGC TTC CTT GCT CTC GAC AAC ACG AAC AAG     288
   Gly Val Gly Asp Val Thr Gly Phe Leu Ala Leu Asp Asn Thr Asn Lys
                   85                  90                  95
   CTT ATC GTC CTC TCT TTC CGT GGC TCA AGA TCT ATA GAG AAC TGG ATC     336
   Leu Ile Val Leu Ser Phe Arg Gly Ser Arg Ser Ile Glu Asn Trp Ile
               100                 105                 110
   GGG AAT CTT AAC TTC GAC TTG AAA GAA ATA AAT GAC ATT TGC TCC GGC     384
   Gly Asn Leu Asn Phe Asp Leu Lys Glu Ile Asn Asp Ile Cys Ser Gly
           115                 120                 125
   TGC AGG GGA CAT GAC GGC TTC ACT TCG TCC TGG AGG TCT GTA GCC GAT     432
   Cys Arg Gly His Asp Gly Phe Thr Ser Ser Trp Arg Ser Val Ala Asp
       130                 135                 140
   ACG TTA AGG CAG AAG GTG GAG GAT GCT GTT CGC GAG CAT CCC GAC TAT     480
   Thr Leu Arg Gln Lys Val Glu Asp Ala Val Arg Glu His Pro Asp Tyr
   145                 150                 155                 160
   CGC GTG GTG TTT ACC GGC CAT AGC CTT GGT GGT GCG CTA GCA ACT GTT     528
   Arg Val Val Phe Thr Gly His Ser Leu Gly Gly Ala Leu Ala Thr Val
                   165                 170                 175
   GCC GGA GCA GAC CTG CGT GGA AAT GGG TAT GAT ATC GAC GTG TTT TCA     576
   Ala Gly Ala Asp Leu Arg Gly Asn Gly Tyr Asp Ile Asp Val Phe Ser
               180                 185                 190
   TAT GGC GCC CCC CGA GTC GGT AAC CGT GCT TTT GCA GAA TTC CTG ACC     624
   Tyr Gly Ala Pro Arg Val Gly Asn Arg Ala Phe Ala Glu Phe Leu Thr
           195                 200                 205
   GTA CAG ACC GGC GGT ACC CTC TAC CGC ATT ACC CAC ACC AAT GAT ATT     672
   Val Gln Thr Gly Gly Thr Leu Tyr Arg Ile Thr His Thr Asn Asp Ile
       210                 215                 220
   GTC CCT AGA CTC CCG CCT CGA GAA TTC GGT TAC AGC CAT TCT AGC CCA     720
   Val Pro Arg Leu Pro Pro Arg Glu Phe Gly Tyr Ser His Ser Ser Pro
   225                 230                 235                 240
   GAG TAC TGG ATC AAA TCT GGA ACA CTA GTC CCC GTC ACC CGA AAC GAT     768
   Glu Tyr Trp Ile Lys Ser Gly Thr Leu Val Pro Val Thr Arg Asn Asp
                   245                 250                 255
   ATC GTG AAG ATA GAA GGC ATC GAT GCC ACC GGC GGC AAT AAC CAG CCT     816
   Ile Val Lys Ile Glu Gly Ile Asp Ala Thr Gly Gly Asn Asn Gln Pro
               260                 265                 270
   AAC ATT CCG GAT ATC CCT GCG CAC CTA TGG TAC TTC GGG TTA ATT GGG        864
   Asn Ile Pro Asp Ile Pro Ala His Leu Trp Tyr Phe Gly Leu Ile Gly
           275                 280                 285
   ACA TGT CTT TAG                                                        876
   Thr Cys Leu  *
       290
(2) INFORMATION FOR SEQ ID NO: 13:
   (i) SEQUENCE CHARACTERISTICS:
      (A) LENGTH: 292 amino acids
      (B) TYPE: amino acid
      (D) TOPOLOGY: linear
   (ii) MOLECULE TYPE: protein
   (xi) SEQUENCE DESCRIPTION: SEQ ID NO: 13:
   Met Arg Ser Ser Leu Val Leu Phe Phe Val Ser Ala Trp Thr Ala Leu
    1               5                  10                  15
   Ala Ser Pro Ile Arg Arg Glu Val Ser Gln Asp Leu Phe Asn Gln Phe
               20                  25                  30
   Asn Leu Phe Ala Gln Tyr Ser Ala Ala Ala Tyr Cys Gly Lys Asn Asn
           35                  40                  45
   Asp Ala Pro Ala Gly Thr Asn Ile Thr Cys Thr Gly Asn Ala Cys Pro
       50                  55                  60
   Glu Val Glu Lys Ala Asp Ala Thr Phe Leu Tyr Ser Phe Glu Asp Ser
   65                  70                  75                  80
   Gly Val Gly Asp Val Thr Gly Phe Leu Ala Leu Asp Asn Thr Asn Lys
                   85                  90                  95
   Leu Ile Val Leu Ser Phe Arg Gly Ser Arg Ser Ile Glu Asn Trp Ile
               100                 105                 110
   Gly Asn Leu Asn Phe Asp Leu Lys Glu Ile Asn Asp Ile Cys Ser Gly
           115                 120                 125
   Cys Arg Gly His Asp Gly Phe Thr Ser Ser Trp Arg Ser Val Ala Asp
       130                 135                 140
   Thr Leu Arg Gln Lys Val Glu Asp Ala Val Arg Glu His Pro Asp Tyr
   145                 150                 155                 160
   Arg Val Val Phe Thr Gly His Ser Leu Gly Gly Ala Leu Ala Thr Val
                   165                 170                 175
   Ala Gly Ala Asp Leu Arg Gly Asn Gly Tyr Asp Ile Asp Val Phe Ser
               180                 185                 190
   Tyr Gly Ala Pro Arg Val Gly Asn Arg Ala Phe Ala Glu Phe Leu Thr
           195                 200                 205
   Val Gln Thr Gly Gly Thr Leu Tyr Arg Ile Thr His Thr Asn Asp Ile
       210                 215                 220
   Val Pro Arg Leu Pro Pro Arg Glu Phe Gly Tyr Ser His Ser Ser Pro
   225                 230                 235                 240
   Glu Tyr Trp Ile Lys Ser Gly Thr Leu Val Pro Val Thr Arg Asn Asp
                   245                 250                 255
   Ile Val Lys Ile Glu Gly Ile Asp Ala Thr Gly Gly Asn Asn Gln Pro
               260                 265                 270
   Asn Ile Pro Asp Ile Pro Ala His Leu Trp Tyr Phe Gly Leu Ile Gly
           275                 280                 285
   Thr Cys Leu  *
       290
(2) INFORMATION FOR SEQ ID NO: 14:
   (i) SEQUENCE CHARACTERISTICS:
      (A) LENGTH: 864 base pairs
      (B) TYPE: nucleic acid
      (C) STRANDEDNESS: single
      (D) TOPOLOGY: linear
   (ii) MOLECULE TYPE: DNA (genomic)
   (vi) ORIGINAL SOURCE:
      (B) STRAIN: *Pseudomonas* sp.
   (ix) FEATURE:
      (A) NAME/KEY: mat_peptide
      (B) LOCATION:1..864
   (ix) FEATURE:
      (A) NAME/KEY: CDS
      (B) LOCATION:1..864
   (xi) SEQUENCE DESCRIPTION: SEQ ID NO: 14:
   TTC GGC TCC TCG AAC TAC ACC AAG ACC CAG TAC CCG ATC GTC CTG ACC       48
   Phe Gly Ser Ser Asn Tyr Thr Lys Thr Gln Tyr Pro Ile Val Leu Thr
    1               5                  10                  15
   CAC GGC ATG CTC GGT TTC GAC AGC CTG CTT GGA GTC GAC TAC TGG TAC       96
   His Gly Met Leu Gly Phe Asp Ser Leu Leu Gly Val Asp Tyr Trp Tyr
               20                  25                  30
   GGC ATT CCC TCA GCC CTG CGT AAA GAC GGC GCC ACC GTC TAC GTC ACC      144
   Gly Ile Pro Ser Ala Leu Arg Lys Asp Gly Ala Thr Val Tyr Val Thr
           35                  40                  45
   GAA GTC AGC CAG CTC GAC ACC TCC GAA GCC CGA GGT GAG CAA CTG CTG      192
   Glu Val Ser Gln Leu Asp Thr Ser Glu Ala Arg Gly Glu Gln Leu Leu
       50                  55                  60
   ACC CAA GTC GAG GAA ATC GTG GCC ATC AGC GGC AAG CCC AAG GTC AAC      240
   Thr Gln Val Glu Glu Ile Val Ala Ile Ser Gly Lys Pro Lys Val Asn
   65                  70                  75                  80
   CTG TTC GGC CAC AGC CAT GGC GGG CCT ACC ATC CGC TAC GTT GCC GCC      288
   Leu Phe Gly His Ser His Gly Gly Pro Thr Ile Arg Tyr Val Ala Ala
                   85                  90                  95
   GTG CGC CCG GAT CTG GTC GCC TCG GTC ACC AGC ATT GGC GCG CCG CAC      336
   Val Arg Pro Asp Leu Val Ala Ser Val Thr Ser Ile Gly Ala Pro His
               100                 105                 110
   AAG GGT TCG GCC ACC GCC GAC TTC ATC CGC CAG GTG CCG GAA GGA TCG      384
   Lys Gly Ser Ala Thr Ala Asp Phe Ile Arg Gln Val Pro Glu Gly Ser
           115                 120                 125
   GCC AGC GAA GCG ATT CTG GCC GGG ATC GTC AAT GGT CTG GGT GCG CTG      432
   Ala Ser Glu Ala Ile Leu Ala Gly Ile Val Asn Gly Leu Gly Ala Leu
       130                 135                 140
   ATC AAC TTC CTT TCC GGC AGC AGT TCG GAC ACC CCA CAG AAC TCG CTG      480
   Ile Asn Phe Leu Ser Gly Ser Ser Ser Asp Thr Pro Gln Asn Ser Leu
   145                 150                 155                 160
   GGC ACG CTG GAG TCA CTG AAC TCC GAA GGC GCC GCA CGG TTT AAC GCC      528
   Gly Thr Leu Glu Ser Leu Asn Ser Glu Gly Ala Ala Arg Phe Asn Ala
                   165                 170                 175
   CGC TTC CCC CAG GGG GTA CCA ACC AGC GCC TGC GGC GAG GGC GAT TAC      576
   Arg Phe Pro Gln Gly Val Pro Thr Ser Ala Cys Gly Glu Gly Asp Tyr
               180                 185                 190
   GTG GTC AAT GGC GTG CGC TAT TAC TCC TGG AGG GGC ACC AGC CCG CTG      624
   Val Val Asn Gly Val Arg Tyr Tyr Ser Trp Arg Gly Thr Ser Pro Leu
           195                 200                 205
   ACC AAC GTA CTC GAC CCC TCC GAC CTG CTG CTC GGC GCC ACC TCC CTG      672
   Thr Asn Val Leu Asp Pro Ser Asp Leu Leu Leu Gly Ala Thr Ser Leu
       210                 215                 220
   ACC TTC GGT TTC GAG GCC AAC GAT GGT CTG GTC GGA CGC TGC AGC TCC      720
   Thr Phe Gly Phe Glu Ala Asn Asp Gly Leu Val Gly Arg Cys Ser Ser
   225                 230                 235                 240
   CGG CTG GGT ATG GTG ATC CGC GAC AAC TAC CGG ATG AAC CAC CTG GAC      768
   Arg Leu Gly Met Val Ile Arg Asp Asn Tyr Arg Met Asn His Leu Asp
                   245                 250                 255
   GAG GTG AAC CAG ACC TTC GGG CTG ACC AGC ATC TTC GAG ACC AGC CCG      816
   Glu Val Asn Gln Thr Phe Gly Leu Thr Ser Ile Phe Glu Thr Ser Pro
               260                 265                 270
   GTA TCG GTC TAT CGC CAG CAA GCC AAT CGC CTG AAG AAC GCC GGG CTC      864
   Val Ser Val Tyr Arg Gln Gln Ala Asn Arg Leu Lys Asn Ala Gly Leu
           275                 280                 285
(2) INFORMATION FOR SEQ ID NO: 15:
   (i) SEQUENCE CHARACTERISTICS:
      (A) LENGTH: 288 amino acids
      (B) TYPE: amino acid
      (D) TOPOLOGY: linear
   (ii) MOLECULE TYPE: protein
   (xi) SEQUENCE DESCRIPTION: SEQ ID NO: 15:
   Phe Gly Ser Ser Asn Tyr Thr Lys Thr Gln Tyr Pro Ile Val Leu Thr
    1               5                  10                  15
   His Gly Met Leu Gly Phe Asp Ser Leu Leu Gly Val Asp Tyr Trp Tyr
               20                  25                  30
   Gly Ile Pro Ser Ala Leu Arg Lys Asp Gly Ala Thr Val Tyr Val Thr
           35                  40                  45
   Glu Val Ser Gln Leu Asp Thr Ser Glu Ala Arg Gly Glu Gln Leu Leu
       50                  55                  60
   Thr Gln Val Glu Glu Ile Val Ala Ile Ser Gly Lys Pro Lys Val Asn
   65                  70                  75                  80
   Leu Phe Gly His Ser His Gly Gly Pro Thr Ile Arg Tyr Val Ala Ala
                   85                  90                  95
   Val Arg Pro Asp Leu Val Ala Ser Val Thr Ser Ile Gly Ala Pro His
               100                 105                 110
   Lys Gly Ser Ala Thr Ala Asp Phe Ile Arg Gln Val Pro Glu Gly Ser
           115                 120                 125
   Ala Ser Glu Ala Ile Leu Ala Gly Ile Val Asn Gly Leu Gly Ala Leu
       130                 135                 140
   Ile Asn Phe Leu Ser Gly Ser Ser Ser Asp Thr Pro Gln Asn Ser Leu
   145                 150                 155                 160
   Gly Thr Leu Glu Ser Leu Asn Ser Glu Gly Ala Ala Arg Phe Asn Ala
                   165                 170                 175
   Arg Phe Pro Gln Gly Val Pro Thr Ser Ala Cys Gly Glu Gly Asp Tyr
               180                 185                 190
   Val Val Asn Gly Val Arg Tyr Tyr Ser Trp Arg Gly Thr Ser Pro Leu
           195                 200                 205
   Thr Asn Val Leu Asp Pro Ser Asp Leu Leu Leu Gly Ala Thr Ser Leu
       210                 215                 220
   Thr Phe Gly Phe Glu Ala Asn Asp Gly Leu Val Gly Arg Cys Ser Ser
   225                 230                 235                 240
   Arg Leu Gly Met Val Ile Arg Asp Asn Tyr Arg Met Asn His Leu Asp
                   245                 250                 255
   Glu Val Asn Gln Thr Phe Gly Leu Thr Ser Ile Phe Glu Thr Ser Pro
               260                 265                 270
   Val Ser Val Tyr Arg Gln Gln Ala Asn Arg Leu Lys Asn Ala Gly Leu
           275                 280                 285